(19) **Europäisches Patentamt**
**European Patent Office**
**Office européen des brevets**

(11) **EP 2 700 934 B1**

(12) **EUROPEAN PATENT SPECIFICATION**

(45) Date of publication and mention
of the grant of the patent:
**14.12.2016  Bulletin 2016/50**

(21) Application number: **12773621.3**

(22) Date of filing: **17.04.2012**

(51) Int Cl.:
*G01N 21/64* (2006.01)    *C12N 15/09* (2006.01)
*C12Q 1/68* (2006.01)    *G01N 15/14* (2006.01)
*G01N 15/00* (2006.01)    *G02B 21/00* (2006.01)

(86) International application number:
**PCT/JP2012/060334**

(87) International publication number:
**WO 2012/144485 (26.10.2012 Gazette 2012/43)**

(54) **METHOD FOR DETECTING A NUCLEIC ACID MOLECULE IN A BIOSAMPLE**

VERFAHREN ZUR ERKENNUNG VON EINEM NUKLEINSÄUREMOLEKÜL IN EINER BIOLOGISCHEN PROBE

PROCÉDÉ DE DÉTECTION D'UNE MOLÉCULE D'ACIDE NUCLÉIQUE DANS UN ÉCHANTILLON BIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.04.2011   JP 2011094293**

(43) Date of publication of application:
**26.02.2014   Bulletin 2014/09**

(73) Proprietor: **Olympus Corporation
Tokyo 192-8507 (JP)**

(72) Inventor: **NISHIKAWA, Kazutaka
Hachioji-shi, Tokyo 192-8507 (JP)**

(74) Representative: **Schicker, Silvia
Wuesthoff & Wuesthoff
Patentanwälte PartG mbB
Schweigerstraße 2
81541 München (DE)**

(56) References cited:
**WO-A1-2009/158451    WO-A1-2010/074265
WO-A1-2011/108369    WO-A1-2012/014778
WO-A1-2012/014778    JP-A- 2004 532 649
JP-A- 2007 006 890    JP-A- 2008 116 440
JP-A- 2010 190 730    JP-A- 2011 002 415
JP-A- 2011 036 150    JP-A- 2011 508 219
KR-A- 20100 078 827    US-A1- 2001 014 455
US-A1- 2003 092 090    US-A1- 2005 287 616
US-A1- 2008 117 421**

EP 2 700 934 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a method for detecting a nucleic acid molecule in a biosample, with use of an optical system which can detect light from a micro region in a solution, such as an optical system of a confocal microscope and a multiphoton microscope.

Priority is claimed on Japanese Patent Application No. 2011-094293, filed April 20, 2011.

BACKGROUND ART

According to the developments in photometric measurement techniques in recent years, detection/measurement of faint light at a single photon or single fluorescent molecule level have become possible by using an optical system of a confocal microscope and a super high sensitive light detection technique capable of photon counting (single photon detection). Thus, various devices or methods of performing the detection of intermolecular interaction or binding/dissociating reaction of biological molecules etc. using the above-mentioned faint light measurement technique, have been proposed. For example, in Fluorescence Correlation Spectroscopy (FCS, see e.g. Patent Documents 1 and 2 and Non-patent Documents 1 to 3), by using an optical system of a laser confocal microscope and a photon counting technique, the measurement is performed on the fluorescence intensity from fluorescent molecules or fluorescently labeled molecules (fluorescent molecules etc.), entering and exiting a micro region in a sample solution (the focal region to which the laser light of the microscope is condensed, called a "confocal volume"). In Fluorescence Correlation Spectroscopy, the information on the speed of the movement, the size, and the concentration of the fluorescent molecules etc. is acquired based on the average dwell time (translational diffusion time) of the fluorescent molecules etc. and the average number of the dwelling molecules in the micro region, determined from the autocorrelation function value of the measured fluorescence intensity. Or, in Fluorescence Correlation Spectroscopy, various phenomena, such as a change of a molecular structure or size, a binding/dissociating reaction, and dispersion/aggregation of molecules, are detected based on the above-mentioned average dwell time of the fluorescent molecules etc. and the average number of the dwelling molecules. Moreover, in Fluorescence Intensity Distribution Analysis (FIDA, e.g. Patent Document 3) or Photon Counting Histogram (PCH, e.g. Patent Document 4), a histogram is produced on the fluorescence intensity of fluorescent molecules etc., entering and exiting a confocal volume, measured similarly to FCS. In Fluorescence Intensity Distribution Analysis, the average value of the characteristic brightness of the fluorescent molecules etc. and the average number of molecules dwelling in the confocal volume are calculated by fitting a statistical model formula to the distribution of the thus produced histogram. In Fluorescence Intensity Distribution Analysis, based on the information thereof, the change of the molecular structure or size, binding/dissociative conditions, and dispersion/aggregation conditions of molecules are estimated. Furthermore, Patent Documents 5 and 6 propose methods of detecting fluorescent substances based on a time progress of a fluorescence signal of a sample solution measured by using an optical system of a confocal microscope. Patent document 7 proposes a signal calculation processing technique for detecting the existences of the fluorescent fine particles in a flow or on a substrate by measuring faint light from fluorescent fine particles flowing through a flow cytometer or fluorescent fine particles fixed on the substrate using a photon counting technique.

Especially, according to the method employing the fluorescence measurement technique of a micro region by using an optical system of a confocal microscope and a photon counting technique, such as FCS and FIDA, the concentration and the amount of the sample required for the measurement may be extremely small (the amount for use in one measurement may be about several tens of $\mu$L at most), as compared with the prior art. Moreover, according to the above-mentioned method, the measuring time is also shortened a lot (in one measurement, a measuring process taking a time of a second order is repeated several times). Thus, these techniques are expected to be a strong tool enabling an experiment or a test at low cost or quickly in comparison with conventional biochemical methods, especially in the following cases:

in cases of conducting an analysis of rare or expensive samples which are often used in the field of medical or biological research and development; and
in cases of conducting a test of a large number of specimens, such as clinical diagnosis of diseases or the screening of bioactive substances.

On the other hand, as a method for detecting a nucleic acid having a specific base sequence, many methods are proposed to investigate the base sequence of a nucleic acid by using an artificially synthesized short chain oligonucleotide such as a probe or a primer. Particularly, many biosamples are present at small amounts in general, like specimens in a clinical test, or the like. Therefore, for analyzing a nucleic acid in a biosample, widely used are methods to amplify nucleic acids contained in the biosample by using a usual molecular biological technique such as PCR, and thereafter or at the same time of amplifying, detecting the target nucleic acid. Moreover, usually, a wide variety of substances other than nucleic acids are contained in a biosample, and some times inhibitory substances against a nucleic acid amplification reaction may be contained. For this reason, for precisely detecting a nucleic acid by using a nucleic acid amplification reaction, it is necessary to selectively extract and collect nucleic acids from the biosample in advance so as to reduce the influences of impurities such as inhibitory substances against the nucleic acid amplification reaction. For example, Patent Document 8 discloses a method in which nucleic acids are extracted from a biosample by a method to collect nucleic acids using a magnetic carrier, and subsequently the nucleic acid of interest is detected from the extracted nucleic acids by using a usual molecular biological technique such as the nucleic acid amplification method. Moreover, Patent Document 9 discloses a method in which, after coagulating blood, the obtained serum is subjected to a nucleic acid amplification reaction so as to remove inhibitory substances against the nucleic acid amplification reaction contained in the blood, and then the nucleic acid of interest is amplified and detected. Besides, Patent Document 10 discloses a method in which blood is diluted and then heated, and the obtained sample is subjected to a nucleic acid amplification reaction so as to detect the nucleic acid of interest.

PRIOR ART DOCUMENTS

PATENT DOCUMENTS

Patent document 1: Japanese Unexamined Patent Application, First Publication No. 2005-098876
Patent document 2: Japanese Unexamined Patent Application, First Publication No. 2008-292371
Patent document 3: Japanese Patent No. 4023523
Patent document 4: PCT International Publication No. WO 2008-080417
Patent document 5: Japanese Unexamined Patent Application, First Publication No. 2007-20565
Patent document 6: Japanese Unexamined Patent Application, First Publication No. 2008-116440
Patent document 7: Japanese Unexamined Patent Application, First Publication No. H04-337446
Patent document 8: Japanese Patent No. 2965131
Patent document 9: Japanese Patent No. 3575633
Patent document 10: Japanese Unexamined Patent Application, First Publication No. 2006-187221

NON-PATENT DOCUMENTS

Non-patent document 1: Masataka Kinjo; "Protein, Nucleic acid, and Enzyme" Vol. 44, No. 9, pages 1431-1438, 1999.
Non-patent document 2: Meyer-Alms; "Fluorescence Correlation Spectroscopy" edt. R. Rigler, Springer, Berlin, pages 204-224, 2000.
Non-patent document 3: Noriko Kato, et al. "Gene & Medicine", Vol. 6, No. 2, pages 271-277, 2002.

WO 2009/158451 A1 discloses a Fluorescence Correlations Spectroscopy (FCS) method for performing melting curve analyses of nucleic acids on a microarray. The method makes use of an apparatus comprising an illumination system, an emissions detection system, a fluid circulation system, a microarray translation stage and a thermal control. The measurement side comprises a fluorescence detection system. Target DNA which has been permanently stained will fluoresce when bound to probes and when floating in solution. The apparatus and method permits detecting and quantifying the amount of perfectly matched target DNA to the probe from the loss of fluorescence due to double stranded DNA melting into single stranded DNA. The FCS arrangement has a pin hole and lens and uses autocorrelation to derive, from the light fluctuations events in which individual species enter or exit the observation volume defined by the confocal microscope objective statistical characteristics of the molecular species.

DISCLOSURE OF INVENTION

PROBLEMS TO BE SOLVED BY THE INVENTION

In the above-mentioned photometric analysis techniques, such as FCS, FIDA, and PCH, generally speaking, the magnitude of chronological fluctuation of the measured fluorescence intensity is computed by a statistical process, and various characteristics of fluorescent molecules etc., entering and exiting a micro region in a sample solution, are determined based on the magnitude of the fluctuation. Thus, in order to obtain a significant result in the above-mentioned photometric analysis technique, it is preferable to prepare the concentration or number density of fluorescent molecules etc. serving as the object of observation in a sample solution so that fluorescent molecules etc. of a number enabling a statistical process will enter and exit a micro region during a one-time measurement period of a length of a second order in an equilibrium. Suitably, it is preferable to prepare so that about one fluorescent molecule etc. will always exist in the micro region. Typically, it is preferable that the concentration of fluorescent molecules etc. is about 1 nM or more, since the volume of a confocal volume is about 1 fL. In other words, when the concentration or number density of particles as the object of observation in a sample solution is much lower than the level enabling a statistical process (for example, much lower than 1 nM), a condition may occur in which an object as the object of observation rarely enters the micro region within the period of the measurement, and accordingly the measurement result of the fluorescence intensity would include a condition in which no object of observation exist at all in the micro region over a long period of time, and also the observable amount of significant fluorescence intensity would decrease. As a result, any significant or precise analysis result can not be obtained in the photometric analysis technique based on the statistical fluctuation of the fluorescence intensity as described above.

In the method of detecting fluorescent substances by using an optical system of a confocal microscope described in Patent Documents 5 and 6, it is disclosed that it is possible to obtain a correlation between the frequency of fluorescence signals having a significant intensity and the number of particles such as fluorescent molecules etc. in a sample, without performing a statistical process of the fluorescence intensity fluctuation as described above. This is because it is possible to determine the presence or absence of a fluorescent molecule etc. serving as the object of observation in a sample, from the presence or absence of generation of a fluorescence signal having a significant intensity in the period of measurement over several seconds. In particular, in Patent Document 6, it is suggested that the generation of a random flow agitating the inside of a sample solution improves the detection sensitivity. However, in these methods, it is only possible to detect the existences of fluorescent molecules etc. entering a micro region at random by diffusion or a random flow, and it is not possible to grasp the behavior of a particle such as a fluorescent molecule etc. in the micro region. For instance, the counting of particles or the quantitative computing of the concentration or number density of particles has not been achieved. Moreover, the technique described in Patent Document 7 is a technique to detect individual existences of fluorescent fine particles in a flow in a flow cytometer or fluorescent fine particles fixed on a substrate, and is not a technique to detect particles, such as molecules or colloids, being dissolved or dispersed in a normal condition in a sample solution. That is, since the technique described in Patent Document 7 is not a technique to detect particles moving at random in a sample solution, it has not been achieved to quantitatively compute out the concentration or number density of particles dissolved or dispersed in a sample solution. Moreover, the technique of patent document 7 includes processes, such as the measurement in a flow cytometer or the treatment of fixing fluorescence particles on a substrate. Thus, it is considered that, in the technique of patent document 7, the amount of the sample necessary for the test should be much larger than those of the cases of photometric analysis techniques, such as FCS, FIDA, and PCH, and also complicated and advanced operational techniques should be requested for a person who conducts the test.

In addition, with a detection method having a sufficient detection sensitivity, it is expected to be possible to detect a nucleic acid of interest directly from a biosample which contains only a very small amount of the nucleic acid of interest serving as the object of detection, without performing a nucleic acid amplification reaction in advance. However, some impurities contained together with nucleic acids in a biosample have autofluorescence. For this reason, in the case of directly detecting a nucleic acid in a biosample with the photometric analysis technique, there is a problem in that nonspecific signals may occur because of various kinds of impurities derived from the biosample. Moreover, there is also a problem in that transmitted light may be blocked by such various kinds of impurities derived from the biosample. The presence of impurities hindering the photometric measurement is a severer problem as the detection sensitivity of the photometric analysis technique is higher. That is to say, in the case of detecting a nucleic acid of interest from a biological body-derived sample such as blood or tissue with a highly sensitive photometric analysis technique, it is very difficult to accurately measure the concentration of the object of the measurement per se because of the impurities other than the object of the measurement which hinder the measurement.

It is an object of the present invention to provide a method for detecting a nucleic acid molecule in a biosample which contains a wide variety of substances. The method provided by the present invention employs a novel photometric analysis technique. This novel photometric analysis technique does not include a statistical process as executed in photometric analysis techniques, such as FCS, FIDA, and PCH. Accordingly, it is possible to detect a condition or a characteristic of particles as the object of observation in a sample solution in which the concentration or number density of the particles as the object of observation is lower than the level that can be treated by these photometric analysis techniques.

MEANS TO SOLVE THE PROBLEMS

The novel photometric analysis technique employed in the detection method provided by the present invention has the following characteristics. That is, as a method for detecting a nucleic acid, there is a method in which a nucleic acid probe which is specifically hybridizable with the nucleic acid molecule is used so as to detect the nucleic acid molecule in a biosample depending on whether or not an associated body with the nucleic acid probe has been formed. In this method, the detection of the associated body including the nucleic acid probe is performed by using the scanning molecule counting method, and therefore the nucleic acid molecule can be detected with good sensitivity even if the concentration of the nucleic acid molecule to be analyzed in the sample is very low. Furthermore, the nucleic acid molecule can be precisely detected by removing biosample-derived impurities in advance prior to the detection of the associated body including the nucleic acid probe.

The above-mentioned scanning molecule counting method is a novel photometric analysis technique proposed by the applicant of this application in Japanese Patent Application No. 2010-044714.

This invention proposes the following means so as to solve the above-mentioned problems.

The method for detecting a nucleic acid molecule according to a first aspect of the present invention comprises a preparing step, an associating step, and a calculating step.

The preparing step is to prepare a sample solution which contains a nucleic acid probe which is specifically hybridizable with the nucleic acid molecule to be analyzed, and a biosample.

The associating step is to associate the nucleic acid molecule with the nucleic acid probe in the sample solution that has been prepared in the preparing step.

The calculating step is to, after the associating step, calculate the number of molecules of the associated bodies including the nucleic acid probe in the sample solution that has been prepared in the preparing step.

Furthermore, the method for detecting a nucleic acid molecule according to the first aspect of the present invention comprises a step for moving the position of a light detection region, a step for detecting fluorescence, a step for individually detecting the associated body, a counting step, and a removing step.

The step for moving the position of a light detection region is to move the position of a light detection region of an optical system of a confocal microscope or a multiphoton microscope in the sample solution in the calculating step. The step for detecting fluorescence is to detect fluorescence emitted from the associated bodies in the light detection region while moving the position of the light detection region of the optical system in the sample solution.

The step for individually detecting the associated body is to individually detect the associated body by individually detecting a light signal from respective associated body, from the detected light.

The counting step is to count the number of the individually detected associated bodies to thereby count the number of the particles having been detected during the moving of the position of the light detection region.

The removing step is to remove proteins from the sample solution before the calculating step. Or, the removing step is to remove proteins from the biosample before the preparing step.

The method for detecting a nucleic acid molecule according to a second aspect of the present invention comprises a step for removing proteins from the sample solution after the associating step and before the calculating step.

The method for detecting a nucleic acid molecule according to a third aspect of the present invention carries out the removal of proteins from the sample solution or the biosample by adsorbing nucleic acid molecules in the concerned sample solution or the concerned biosample to an inorganic support, and thereafter eluting the adsorbed nucleic acids from the inorganic support.

According to a fourth aspect of the present invention, the biosample is treated with a proteinase after being collected from a biological body.

According to a fifth aspect of the present invention, the nucleic acid probe is labeled with a fluorescent substance.

According to a sixth aspect of the present invention, the nucleic acid probe is labeled with a fluorescent substance. In the preparing step, a fluorescent double stranded nucleic acid-binding substance is further contained in the sample solution. Either one of the fluorescent substance labeling the nucleic acid probe and the fluorescent double stranded nucleic acid-binding substance is a fluorescent substance serving as an energy donor in a fluorescence energy transfer phenomenon, and the other one is a substance serving as an energy acceptor in the fluorescence energy transfer phenomenon. In the calculating step, the fluorescence emitted from the associated body including the nucleic acid probe is fluorescence emitted from the fluorescence energy transfer phenomenon occurring between the fluorescent substance labeling the nucleic acid probe and the fluorescent double stranded nucleic acid-binding substance.

According to a seventh aspect of the present invention, the nucleic acid probe is bound with a fluorescent substance serving as an energy donor and a substance serving as an energy acceptor so that fluorescence energy transfer occurs in a state in which the nucleic acid probe solely exists, and the fluorescence energy transfer does not occur in a state in which the nucleic acid probe forms an associated body with another single stranded nucleic acid molecule. The fluorescence emitted from the concerned associated body including the nucleic acid probe is fluorescence emitted from the fluorescent substance serving as an energy donor.

According to an eighth aspect of the present invention, the position of the light detection region is moved at a predetermined speed, in the step for moving the position of the light detection region.

According to a ninth aspect of the present invention, the position of the light detection region is moved at a speed higher than the speed of diffusional movement of the associated bodies, in the step for moving the position of the light detection region.

In the method for detecting a nucleic acid molecule according to a tenth aspect of the present invention, it is detected that one associated body has entered the light detection region, based on the shape of a chronologically detected light signal, in the step for individually detecting the associated body by individually detecting a light signal from respective associated body, from the detected light.

According to an eleventh aspect of the present invention, the sample solution contains one or more types of substances selected from the group consisting of a surfactant, formamide, dimethyl sulfoxide, and urea.

In the method for detecting a nucleic acid molecule according to a twelfth aspect of the present invention, the associating step is to, after denaturing the nucleic acid molecules in the sample solution by setting the temperature of the sample solution having been prepared in the preparing step at 70°C or higher, associate the nucleic acid molecules in the sample solution by lowering the liquid temperature of this sample solution at a cooling rate of 0.05°C/sec. or quicker.

According to a thirteenth aspect of the present invention, the nucleic acid probe comprises a binding of two or more molecules selected from the group consisting of DNA, RNA, and a nucleic acid analog.

EFFECT OF THE INVENTION

In the scanning molecule counting method employed in the above-mentioned method for detecting a nucleic acid molecule, no statistical process such as computing the fluorescence intensity fluctuation is executed. Therefore, according to the above-mentioned method for detecting a nucleic acid molecule, the nucleic acid molecule to be analyzed can be detected even if the nucleic acid molecule exists at an extremely small amount in a sample.

In the above-mentioned method for detecting a nucleic acid molecule, additionally, proteins derived from the biosample are removed from the sample solution before conducting the detection of the associated body of the nucleic acid probe and the nucleic acid molecule to be analyzed in the biosample by means of the scanning molecule counting method. For this reason, the nucleic acid molecule to be analyzed can be more precisely detected by the scanning molecule counting method.

BRIEF DESCRIPTION OF THE DRAWINGS

FIG. 1A is a schematic diagram of the internal structure of a photometric analysis device for the scanning molecule counting method in an embodiment of the present invention.

FIG. 1B is a schematic diagram of a confocal volume (an observation region of a confocal microscope) of the photometric analysis device of FIG. 1A.

FIG. 1C is a schematic diagram of a mechanism for changing the direction of a reflective mirror 7 of the photometric analysis device of FIG. 1A to move the position of a light detection region in a sample solution.

FIG. 2A is a schematic diagram explaining the principle of light detection by the photometric analysis technique for the scanning molecule counting method.

FIG. 2B is a schematic diagram of the chronological change of the light intensity measured by the photometric analysis technique for the scanning molecule counting method.

FIG. 3A is a drawing of a model in the case that particles as the object of observation are passing across a light detection region owing to Brownian motion.

FIG. 3B is a diagram showing an example of the chronological change of the photon counts (light intensity) in the case of FIG. 3A.

FIG. 4A is a drawing of a model in the case that particles as the object of observation are passing across a light detection region by moving the position of the light detection region in a sample solution at a speed higher than the speed of diffusional movement of the particles as the object of observation.

FIG. 4B is a diagram showing an example of the chronological change of the photon counts (light intensity) in the case of FIG. 4A.

FIG. 5 is a flow chart showing the procedure of processes for counting the particles from the chronological change of the photon counts (light intensity) measured by the scanning molecule counting method.

FIG. 6A is a drawing explaining an example of the signal processing step of the detected signals in the procedure of processes for counting the particles from the chronological change of the photon counts (light intensity) measured by the scanning molecule counting method.

FIG. 6B are drawings explaining an example of the signal processing step of the detected signals in the procedure of processes for counting the particles from the chronological change of the photon counts (light intensity) measured by the scanning molecule counting method.

FIG. 7 shows an example of the photon count data measured by the scanning molecule counting method (bar graph), a curve obtained by smoothing the data (dotted line), and Gauss functions fitted on the peak existence regions (solid line). In the drawing, the signals appended as "noise" are disregarded as signals derived from a noise or a contaminant.

FIG. 8 is a graph showing the counted number of peaks in respective sample solutions in Test Example 1 of the present invention.

FIG. 9 is a graph showing the relations between the concentration of the associated body in the sample solution and the counted number of peaks in respective sample solutions in Example 1 of the present invention.

FIG. 10 is a graph showing the counted number of peaks in respective sample solutions in Example 2 of the present invention.

BEST MODE FOR CARRYING OUT THE INVENTION

Firstly, the scanning molecule counting method is described. In the scanning molecule counting method, while scanning the inside of a sample solution with a micro region, light emitted from a luminescent particle dispersed and moving at random in the sample solution (hereunder, referred to as "luminescent particle") in the micro region, is detected when the luminescent particle passes across the inside of the micro region. That is, the scanning molecule counting method is a technique which enables, by individually detecting luminescent particles in the sample solution one by one, the counting of the luminescent particles, and the acquisition of the information about the concentration or number density of the luminescent particles in the sample solution. In the scanning molecule counting method, similarly to the photometric analysis techniques such as FIDA, the amount of the sample required for the measurement may be extremely small (e.g., several tens of $\mu$L). Moreover, in the scanning molecule counting method, the measuring time is short, and furthermore, the characteristics of the luminescent particles, such as its concentration or number density, can be quantitatively detected even when the concentration or number density thereof are lower than the cases of the photometric analysis techniques such as FIDA.

The luminescent particle means a particle in which the particle serving as the object of observation and the luminescent probe are bound or associated. The term "luminescent probe" is a substance having a property to be bound to or associated with the particle serving as the object of observation, as well as to emit light (usually, a molecule or an aggregate thereof). The luminescent particle is typically a fluorescence particle, although it may be a particle which emits light by phosphorescence, chemiluminescence, bioluminescence, light scattering, etc. The nucleic acid probe used in the method for detecting a nucleic acid molecule of this embodiment corresponds to the luminescent probe.

In the embodiment of the present invention, the "light detection region" of an optical system of a confocal microscope or a multiphoton microscope is a micro region where light is detected in these microscopes. The light detection region corresponds to the region to which illumination light is condensed when the illumination light is given from an object lens. Note that, the above-mentioned region is determined in accordance with the spatial relationship of an object lens and a pinhole especially in a confocal microscope.

The detection of light is sequentially performed while moving the position of the light detection region in the sample solution. That is, the detection of light is performed while scanning the inside of the sample solution with the light detection region. Then, when the moving light detection region includes a luminescent probe bound to or associated with a randomly moving particle, light from the luminescent probe is detected. By so doing, the existence of one particle is detected. There may be a case, depending on the embodiment of the experiment, where the luminescent probe has been dissociated from a particle desired to be detected at the time of the light detection, after once being bound to the particle. Then, the light signal from each luminescent probe is individually detected with sequentially detected light. By so doing, the existence of a particle (bound to the luminescent probe) is individually and sequentially detected one by one, and various information on the condition of the particle within the solution are acquired. Concretely, for example, in the above-mentioned structure, the number of the particles detected during the moving of the position of the light detection region may be counted by counting the number of the individually detected particles (the counting of particles). According to the above-mentioned structure, by combining the number of the particles and the moving amount of the position of the light detection region, the information on the number density or concentration of the particles in the sample solution can be acquired. Especially, according to an arbitrary method (for example, a method to determine the whole volume of the moving track of the position of the light detection region by moving the position of the light detection region at a predetermined speed) etc., the number density or concentration of the particles can be concretely computed. Of course, instead of determining directly the absolute number density value or concentration value, the structure may also be designed to compute out the relative ratio of the number density or concentration to a plurality of sample solutions or a standard sample solution serving as a reference of a concentration or a number density. Moreover, the scanning molecule counting method is designed to move the position of the light detection region by changing the optical path of the optical system. For this reason, the light detection region moves quickly, and substantially no mechanical vibration nor hydrodynamic action will be generated in the sample solution. Therefore, the light can be measured under a stable condition where the particles as the object of detection are not influenced by such a mechanical action (if a vibration or flow is applied to the inside of the sample solution, the physical characteristics of the particles may be changed). Moreover, since there is no need of a structure to make the sample solution circulate, the measurement and the analysis are possible with an extremely small amount (about one to several tens of $\mu$L) of the sample solution similarly to the cases of FCS, FIDA, and the like.

In the above-mentioned step of individually detecting the particle, the judgment from the sequentially detected light signals regarding whether or not a luminescent probe bound to one particle has entered the light detection region may be done based upon the shape of a chronologically detected light signal. Note that, in this embodiment, the term "luminescent probe bound to one particle" includes the following cases:

a case where one luminescent probe is bound to one particle;
a case where a plurality of luminescent probes are bound to one particle; and
a case where luminescent probe(s) is/are dissociated from one particle after being bound to the particle, depending on the embodiment.

In this embodiment, typically, the structure may be designed to detect that a luminescent probe bound to one particle has entered the light detection region when a light signal having an intensity greater than a predetermined threshold value is detected.

Moreover, in the above-mentioned step of moving the position of the light detection region, the speed of moving the position of the light detection region in the sample solution may be appropriately modified based on the characteristic of the luminescent probe bound to the particle, or the number density or concentration in the sample solution. As understood by ones ordinarily skilled in the art, the manner of light detected from the luminescent probe bound to the particle may be different depending on the characteristic, or the number density or concentration in a sample solution. Especially, when the speed of moving the light detection region is higher, the amount of light obtained from the luminescent probe bound to one particle will be reduced. Therefore, it is preferable to appropriately modify the speed of moving the light detection region so that the light from the luminescent probe bound to one particle can be measured with good precision or good sensitivity.

Furthermore, in the step of moving the position of the light detection region, the speed of moving the position of the light detection region in the sample solution is preferably set to be higher than the speed of diffusional movement (the average moving speed of particles owing to the Brownian motion) of the luminescent probes bound to the particles serving as the object of detection (that is, associated bodies including the nucleic acid probe in the method for detecting a nucleic acid molecule of this embodiment). As explained above, in the scanning molecule counting method, light emitted from a luminescent probe bound to one particle is detected when the light detection region passes through the position where the luminescent probe bound to one particle exists, thereby detecting the luminescent probe individually. However, if the luminescent probe bound to the particle moves at random owing to the Brownian motion in the solution to thereby enter and exit the light detection region multiple times, the light signal (light signal showing the existence of the particle desired to be detected) will be detected from one luminescent probe multiple times, which makes it difficult to establish a correspondence between the detected light signal and the existence of one particle desired to be detected. Therefore, as described above, the speed of moving the light detection region is set higher than the speed of diffusional movement of the luminescent probes bound to the particles. Concretely, the speed of moving the light detection region is set higher than the speed of diffusional movement of the associated bodies including the nucleic acid probe. By so doing, it becomes possible to establish a correspondence between a luminescent probe bound to one particle and one light signal (light signal showing the existence of one particle). Since the speed of diffusional movement is different depending upon the luminescent probe bound to the particle, it is preferable to appropriately modify the speed of moving the light detection region according to the characteristics (especially, the diffusion constant) of the luminescent probe bound to the particle as described above.

The optical path of the optical system for moving the position of the light detection region may be achieved in an arbitrary way.

For example, the optical path may be designed to be changeable by using a galvanomirror employed in a laser scan type light microscope so that the position of the light detection region can be changed. The movement locus of the position of the light detection region may be set arbitrarily. For example, the movement locus of the position of the light detection region is selectable from circular, elliptical, rectangular, straight, and curvilinear ones.

The light detecting mechanism of the scanning molecule counting method is designed to detect light from a light detection region in a confocal microscope or a multiphoton microscope similarly to the cases of photometric analysis techniques such as FIDA. For this reason, the amount of a sample solution for use in the scanning molecule counting method may be extremely small similarly to that for photometric analysis techniques such as FIDA. However, no statistical process such as computing the fluorescence intensity fluctuation is executed in the scanning molecule counting method. Therefore, the photometric analysis technique of the scanning molecule counting method is applicable to a sample solution in which the number density or concentration of particles is much lower than the level required for the photometric analysis techniques such as FIDA.

Moreover, since the scanning molecule counting method is designed to individually detect each of the particles dispersed or dissolved in a solution, it is possible, by using the information, to quantitatively count the particles, calculate the concentration or number density of the particles in the sample solution, or acquire the information about the concentration or number density. That is, in the scanning molecule counting method, the particles are detected one by one by establishing a one-on-one correspondence between a particle passing through the light detection region and a detected light signal. For this reason, the counting of particles dispersed and moving at random in a solution becomes possible, and also it becomes possible to determine the concentration or number density of particles in a sample solution more precisely as compared with the conventional art. Actually, according to the method for detecting a nucleic acid molecule of this embodiment to determine the particle concentration by individually detecting associated bodies including a nucleic acid probe etc. and counting the number thereof, the nucleic acid molecule can be identified even if the concentration of these associated bodies in the sample solution is much lower than the concentration which can be determined based upon the fluorescence intensity measured by a fluorescence spectrophotometer or a plate reader.

Furthermore, in the manner of scanning the inside of a sample solution with a light detection region by changing the optical path of an optical system, the inside of the sample solution is observed uniformly or under a condition where the sample solution is mechanically stable without applying a mechanical vibration nor a hydrodynamic action to the sample solution. For this reason, the reliability of the quantitative detection result is improved as compared with e.g., the case in which a flow is generated in a sample. This is because, when a flow is given to the sample, it is difficult to give an always uniform flow speed and the device structure becomes complicated. It is also because, when a flow is given to the sample, the required amount of the sample largely increases, and the particle, the luminescent probe, the combined body thereof, or another substance, in the solution may be deteriorated or denaturalized by the hydrodynamic action owing to the flow. Moreover, the measurement can be carried out under a condition where there are no influences or artifacts due to dynamic action against the particles serving as the object of detection in the sample solution (associated bodies including a nucleic acid probe etc. in this embodiment).

<The structure of a photometric analysis device for the scanning molecule counting method>

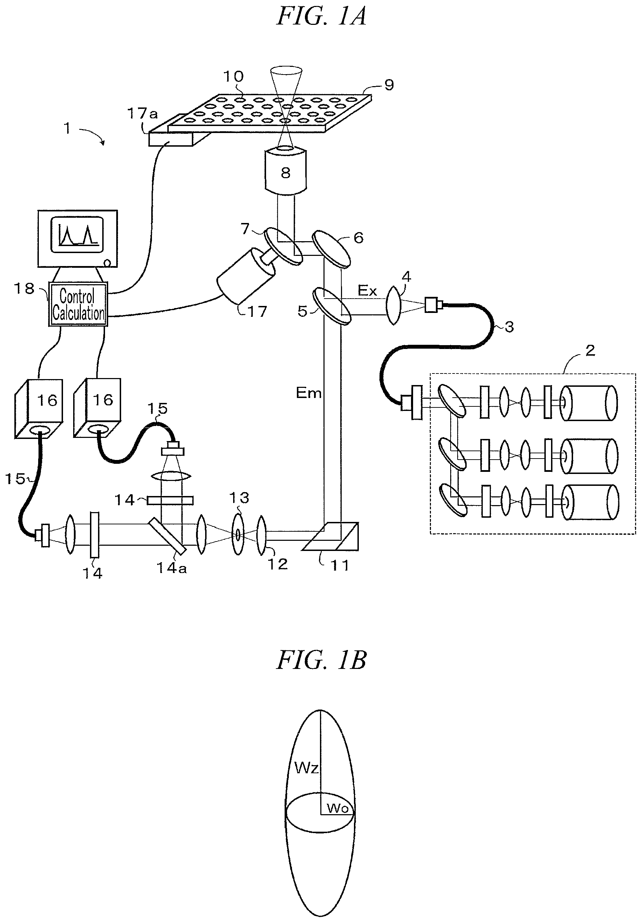

In the basic structure, the scanning molecule counting method can be realized with a photometric analysis device configured by combining an optical system of a confocal microscope and a photodetector as schematically illustrated in FIG. 1A, with which FCS, FIDA, etc. can be performed. Referring to FIG. 1A, the photometric analysis device 1 consists of an optical system 2-17 and a computer 18 for acquiring and analyzing data as well as controlling the operation of each part in the optical system. The optical system of the photometric analysis device 1 may be the same as the optical system of a usual confocal microscope. In this optical system, laser light emitted from a light source 2 and transmitted through the inside of a single mode fiber 3 (Ex) forms light diverging to be radiated at an angle decided by an inherent NA at the emitting end of the fiber, and forms a parallel beam with a collimator 4. This parallel beam is reflected on a dichroic mirror 5 and reflective mirrors 6 and 7, and enters an object lens 8. Typically, above the object lens 8 is placed a sample container or a micro plate 9 having wells 10 arranged therein, to which one to several tens of $\mu$L of a sample solution is dispensed. The laser light emitted from the object lens 8 is focused in the sample solution in the sample container or well 10, forming a region having a strong light intensity (excitation region). In the sample solution, particles serving as the object of observation and luminescent probes to be bound to these particles are dispersed or dissolved. Typically, molecules attached with a light emitting label such as a fluorescent dye are dispersed or dissolved. When a particle which has been bound to or associated with a luminescent probe (a luminescent probe dissociated from a particle after once being bound to the particle in the embodiment of the experiment) enters the excitation region, the luminescent probe is excited and emits light during the period. The emitted light (Em) passes through the object lens 8 and the dichroic mirror 5, is reflected on the mirror 11, is condensed by a condenser lens 12, passes through the pinhole 13, and transmits through a barrier filter 14. When the light (Em) passes through the barrier filter 14, light components only in a specific wavelength band region are selected. Then, the light having the selected light components only in a specific wavelength band region is introduced into a multimode fiber 15, reaching to a photodetector 16. The light reaching to the photodetector 16 is converted into chronological electric signals, and thereafter inputted into the computer 18, where the processes for photometric analyses are executed in manners explained later. In the above-mentioned structure, the pinhole 13 is located at a conjugate position of the focal position of the object lens 8. Thereby, as schematically shown in FIG. 1B, only the light emitted from the focal region of the laser light, i.e., the excitation region, passes through the pinhole 13 while the light from regions other than the excitation region is blocked. The focal region of the laser light illustrated in FIG. 1B is a light detection region in this photometric analysis device, whose effective volume is usually about 1-10 fL, which is called as "confocal volume". Typically, the focal region of the laser light is spread in accordance with a Gaussian type or Lorentz type distribution having the peak of the light intensity at the center of the region. Furthermore, the effective volume of the focal region of the laser light is a volume of an approximate ellipsoid bordering a surface where the light intensity is reduced to $1/e^2$ of the peak intensity.

Moreover, light from one combined body of the particle and the luminescent probe, or the luminescent probe, for example, faint light from one or several fluorescent dye molecule(s), is detected in the scanning molecule counting method. For this reason, preferably, a super high sensitive photodetector, usable for the photon counting, is used for the photodetector 16. Moreover, on the stage (not shown) of the microscope may be provided a stage position changing apparatus 17a for moving the horizontal position of the micro plate 9. This stage position changing apparatus 17a is used in order to change the well 10 for observing the particles etc. The operation of the stage position changing apparatus 17a may be controlled by the computer 18. According to this structure, quick measurement can be achieved even if a plurality of specimens are present.

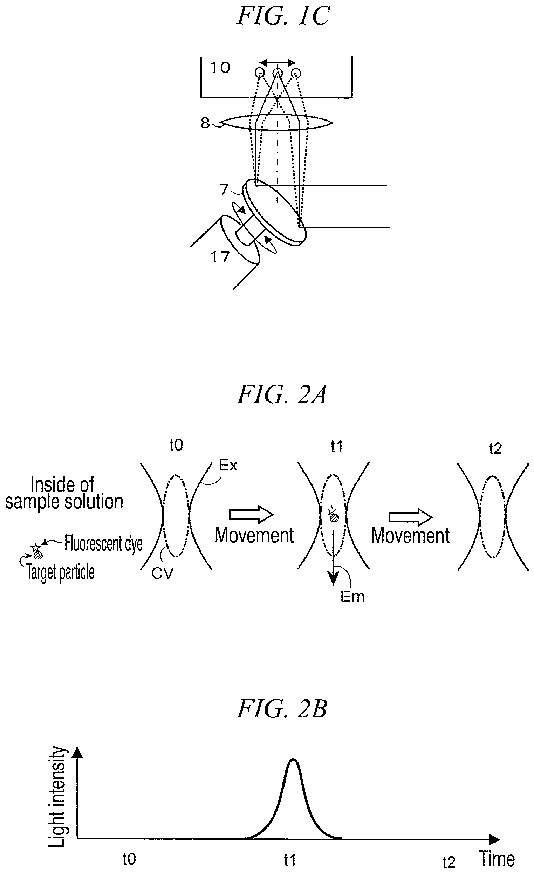

Furthermore, in the optical system of the above-mentioned photometric analysis device is provided a mechanism for changing the optical path of the optical system to scan the inside of the sample solution with the light detection region. In other words, there is provided a mechanism for moving the position of the focal region (i.e., the light detection region) within the sample solution. For the above-mentioned mechanism for moving the position of the light detection region, for example, as schematically illustrated in FIG. 1C, a mirror deflector 17 for changing the direction of the reflective mirror 7 may be employed. The above-mentioned mirror deflector 17 may be the same as that of a galvanomirror device equipped on a usual laser scan type microscope. Moreover, in order to attain a desired moving pattern of the position of the light detection region, the mirror deflector 17 is driven in harmony with the light detection of the photodetector 16 under the control of the computer 18. The movement locus of the position of the light detection region may be arbitrarily selected from circular, elliptical, rectangular, straight, and curvilinear ones, or a combination of these. Moreover, the program in the computer 18 may be designed so that various moving patterns can be selected. Note that, although not illustrated, the position of the light detection region may also be designed to be movable in the vertical direction by moving the object lens 8 up and down. As noted, according to the structure of changing the optical path of the optical system to move the position of the light detection region instead of moving the sample solution, substantially no mechanical vibration or hydrodynamic action will be generated in the sample solution. Consequently, it becomes possible to eliminate the influence of a dynamic action on the object of observation, achieving a stable measurement.

In the case that a combined body of a particle and a luminescent probe, or a luminescent probe, emits light by multiple photon absorption, the above-mentioned optical system is used as a multiphoton microscope. In that case, since the light is emitted only from the focal region of the excitation light (light detection region), the pinhole 13 may be removed. Moreover, in the case that a combined body of a particle and a luminescent probe, or a luminescent probe, emits light owing to a chemiluminescence or bioluminescence phenomenon without excitation light, the optical system 2-5 for generating excitation light may be omitted. In a case that a combined body of a particle and a luminescent probe, or a luminescent probe, emits light owing to phosphorescence or scattered light, the above-mentioned optical system of the confocal microscope is used as it is. Furthermore, in the photometric analysis device 1, as shown in the drawing, a plurality of excitation light sources 2 may be provided. Moreover, the photometric analysis device 1 may be designed so that the wavelength of the excitation light can be selected appropriately in accordance with the excitation wavelength of a combined body of a particle and a luminescent probe or a luminescent probe. Similarly, a plurality of photodetectors 16 may also be provided. Moreover, in a case that the sample contains a plurality of kinds of combined bodies of particles and luminescent probes, or luminescent probes, whose wavelengths differ from each other, the plurality of photodetectors 16 may be configured so that the respective lights from them can be detected separately in accordance with the wavelengths.

<The principle of the photometric analysis technique of the scanning molecule counting method>

Spectral analysis techniques, such as FIDA, are advantageous from the point that the required amount of the sample is extremely small and a test can be executed promptly as compared with the conventional biochemical analytical techniques. However, in the spectral analysis techniques such as FIDA, the concentration and characteristics of the particles as the object of observation are principally calculated based on the fluorescence intensity fluctuation. Therefore, in order to obtain highly precise measurement results, the concentration or number density of the particles as the object of observation in a sample solution should be at a level where about one particle as the object of observation always exists within the light detection region CV during the fluorescence intensity measurement so that a significant light intensity (photon count) can be always detected in the period of the measurement. If the concentration or number density of the particles as the object of observation is lower than that (for example, at the level where the particle as the object of observation rarely enters the light detection region CV), a significant light intensity (photon count) would appear only in a part of the period of the measurement, and thus, highly precise calculation of the light intensity fluctuation would become difficult. Moreover, if the concentration of the particles as the object of observation is much lower than the level where about one particle as the object of observation always exists within the light detection region during the measurement, the calculation of the light intensity fluctuation would be easily influenced by the background, and the period of the measurement should be long in order to obtain a significant quantity of the light intensity data sufficient for the calculation. On the other hand, in the scanning molecule counting method, it is possible to detect the characteristics of the particles as the object of observation, such as its number density or concentration, even if the concentration of the particles as the object of observation is lower than the level required in the spectral analysis techniques such as FIDA.

In the photometric analysis technique of the scanning molecule counting method, briefly speaking, the process to be executed is to change the optical path by driving the mechanism for moving the position of the light detection region (mirror deflector 17). As schematically drawn in FIG. 2, light detection is performed while moving the position of the light detection region CV in a sample solution, that is, while scanning the inside of the sample solution with the light detection region CV.

By so doing, for example, as shown in FIG. 2A, during the moving of the light detection region CV (in the drawing, time t0-t2), when the light detection region CV passes through a region where one particle (in the drawing, a fluorescent dye as a luminescent probe is bound thereto) exists (t1), a significant light intensity (Em) is detected as drawn in FIG. 2B. In that way, by executing the moving of the position of the light detection region CV and the light detection as described above and detecting one by one each significant light intensity appearing as illustrated in FIG. 2B during the period, the particle bound to the luminescent probe is detected individually. By counting the number of the individually detected particles mentioned above, the information about the number, concentration, or number density of the particles existing in the measured region can be acquired. In the principle of this photometric analysis technique of the above-mentioned scanning molecule counting method, since no statistical calculation process, such as the calculation of the fluorescence intensity fluctuation, is conducted and the particles are detected one by one, the information about the concentration or number density of particles can be acquired even with a sample solution whose concentration of the particles to be observed is too low to perform a sufficiently precise analysis in FIDA or the like.

Moreover, according to the method of individually detecting particles in a sample solution and counting them, like in the scanning molecule counting method, it is possible to measure a lower concentration than the case of measuring the concentration of fluorescently labeled particles from the fluorescence intensity measured with a fluorescence spectrophotometer or a plate reader. In the case of measuring the concentration of certain fluorescently labeled particles with a fluorescence spectrophotometer or a plate reader, usually, it is assumed that the fluorescence intensity is proportional to the concentration of the fluorescently labeled particles. However, in that case, when the concentration of the fluorescently labeled particles is significantly low, the amount of noise signals becomes large relative to the amount of signals of the light emitted from the fluorescently labeled particles (deterioration of the S/N ratio), and the proportionality relation between the concentration of the fluorescently labeled particles and the amount of light signals collapses, which deteriorates the precision of the determined concentration value. On the other hand, in the scanning molecule counting method, noise signals are eliminated from the detected result in the step of detecting the signals corresponding to the respective particles from the detected light signals, and the concentration is calculated by counting only the signals corresponding to the respective particles. For this reason, the scanning molecule counting method is capable of detecting a lower concentration than the case of detecting the concentration under the assumption that the fluorescence intensity is proportional to the concentration of the fluorescently labeled particles.

In addition, in the case that a plurality of luminescent probes are bound to one particle as the object of observation, according to the method for individually detecting particles in a sample solution and counting the number thereof like in the scanning molecule counting method, the precision of the measurement of the particle concentration at a higher particle concentration is improved better than conventional methods for determining the concentration under the assumption that the fluorescence intensity is proportional to the concentration of the fluorescently labeled particles. In the case that a plurality of luminescent probes are bound to one particle as the object of observation, when a certain amount of the luminescent probes have been added to the sample solution, the number of the luminescent probes to be bound to the particles relatively decreases as the concentration of the particles as the object of observation increases. In that case, the fluorescence intensity per one particle as the object of observation decreases, and therefore the proportionality relation between the concentration of the fluorescently labeled particles and the amount of light collapses, which deteriorates the precision of the determined concentration value. On the other hand, in the scanning molecule counting method, the influence of the decrease of the fluorescence intensity per one particle is small in the step of detecting the signals corresponding to the respective particles from the detected light signals, and the concentration is calculated from the number of the particles. For this reason, the scanning molecule counting method is capable of detecting a higher concentration than the case of detecting the concentration under the assumption that the fluorescence intensity is proportional to the concentration of the fluorescently labeled particles.

<Measurement of the light intensity of a sample solution by the scanning molecule counting method>

The measurement of the light intensity in the photometric analysis of the scanning molecule counting method may be executed in the same manner as that of the measurement process of the light intensity in FCS or FIDA, except for driving the mirror deflector 17 to move the position of the light detection region within the sample solution (scanning inside of the sample solution) during the measurement. In the operation process, typically, the following procedures are performed. That is, a sample solution is dispensed into the well(s) 10 of the micro plate 9 and placed on the stage of the microscope. Thereafter, a user inputs a command of starting a measurement into the computer 18. The computer 18, into which the command has been input, illuminates the light detection region in the sample solution with the excitation light and starts to measure the light intensity, according to the programs stored in a storage device (not shown). These programs include a procedure for changing the optical path in order to move the position of the light detection region in the sample solution, and a procedure for detecting light from the light detection region during the moving of the position of the light detection region. During the above-mentioned measurement, under the control of the operation process of the computer 18 according to the programs, the mirror deflector 17 drives the mirror 7 (galvanomirror) to move the position of the light detection region in the well 10. Simultaneously with this, the photodetector 16 sequentially converts the detected light into an electric signal and transmits it to the computer 18. The computer 18 generates the chronological light intensity data from the transmitted light signals and stores it in an arbitrary manner. Note that, the photodetector 16 is typically a super high sensitive photodetector which can detect an arrival of a single photon. The detection of light is the photon counting sequentially executed for every predetermined unit time (BINTIME) over a predetermined period of time. For example, the detection of light is executed in the manner of measuring the number of photons which arrive at the photodetector every 10 micro seconds. Moreover, the chronological light intensity data may be chronological photon count data.

The speed of moving the position of the light detection region during the measurement of the light intensity may be a predetermined speed which is arbitrarily set, for example, experimentally or in order to meet the purpose of the analysis. In a case of acquiring the information on the number density or concentration based on the number of detected particles as the object of observation, since the size or volume of the region through which the light detection region has passed is required, the moving of the position of the light detection region is executed in a manner enabling the grasping of the moving distance. Note that, the interpretation of the measurement result will be easy if the elapsed time during the measurement is proportional to the moving distance of the position of the light detection region. For this reason, basically, it is preferable that the moving speed is constant, although the speed is not to be limited thereto.

By the way, regarding the speed of moving the position of the light detection region, in order to quantitatively and precisely execute the individual detection of the particles as the object of observation from the measured chronological light intensity data or the counting of the number of the particles as the object of observation, it is preferable to set the moving speed to a value higher than the moving speed in the random motion, i.e., Brownian motion of the particle as the object of observation. More strictly, the particle as the object of observation is a combined body of a particle and a luminescent probe, or a luminescent probe having been freed from a particle by decomposition after being bound thereto.

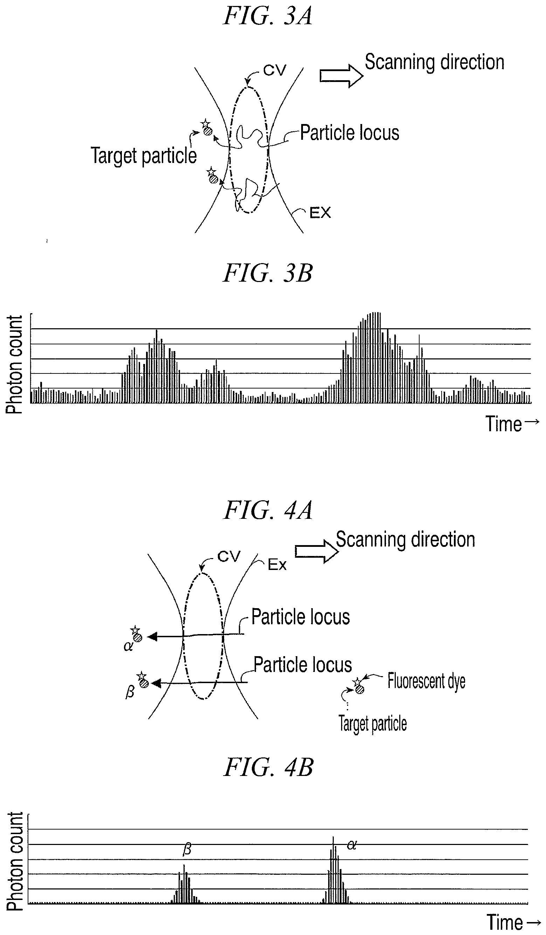

Since the particle as the object of observation in the photometric analysis technique of the scanning molecule counting method is a particle dispersed or dissolved in a solution and moving freely at random, the position of the particle as the object of observation moves with time owing to the Brownian motion. Thus, when the speed of moving the position of the light detection region is slower than the movement of a particle owing to the Brownian motion, the particle moves at random in the region as schematically drawn in FIG. 3A. By so doing, the light intensity changes at random as shown in FIG. 3B, which makes it difficult to specify a significant light intensity change corresponding to each particle as the object of observation. Note that, as already noted, the excitation light intensity in the light detection region is reduced from the peak at the center of the region toward its outside.

Then, suitably, as drawn in FIG. 4A, it is preferable to set so that a particle passes across the light detection region in an approximately straight line. Thereby, the profile of the change of the light intensity corresponding to each particle becomes almost uniform in the chronological light intensity data as illustrated in FIG. 4B, and the speed of moving the position of the light detection region is set to be higher than the average moving speed of the particles owing to the Brownian motion (speed of diffusional movement) so that the correspondence between each particle as the object of observation and the light intensity can be easily specified. Note that, when a particle passes through the light detection region in an approximately straight line, the profile of the change of the light intensity is similar to the excitation light intensity distribution.

Concretely, the time $\Delta t$ required for a particle as the object of observation (more strictly, a combined body of a particle and a luminescent probe, or a luminescent probe having been freed from a particle by decomposition after being bound thereto) having a diffusion coefficient D to pass through the light detection region of radius Wo (confocal volume) by the Brownian motion is given from the expression of the relation of mean-square displacement:

$$(2Wo)^2 = 6D \cdot \Delta t \qquad (1)$$

therefore:

$$\Delta t = (2Wo)^2 / 6D \qquad (2)$$

and thus, the speed of the particle as the object of observation moving by the Brownian motion (speed of diffusional movement) Vdif, becomes approximately

$$Vdif = 2Wo/\Delta t = 3D/Wo \qquad (3)$$

Then, with reference to the above-mentioned Vdif, the speed of moving the position of the light detection region may be set to a sufficiently higher value than Vdif. For example, when the diffusion coefficient of the particle as the object of observation is expected to be about $D = 2.0 \times 10^{-10}$ m$^2$/s, Vdif will be $1.0 \times 10^{-3}$ m/s, supposing Wo is about 0.62 $\mu$m, and therefore, the speed of moving the position of the light detection region may be set to about 10 times thereof at 15 mm/s. Note that, when the diffusion coefficient of the particle as the object of observation is unknown, an appropriate speed of moving the position of the light detection region may be determined by repeating preliminary experiments with setting various moving speeds of the position of the light detection region in order to find the condition that the profile of the change of the light intensity becomes an expected profile (typically, similar to the excitation light intensity distribution).

<Analysis of light intensity by the scanning molecule counting method>

When the chronological light intensity data of a sample solution are obtained by the above-mentioned processes, an analysis of the light intensity as described below may be executed in the computer 18 through the processes in accordance with the programs stored in a storage device.

(i) Detection of one particle as the object of observation

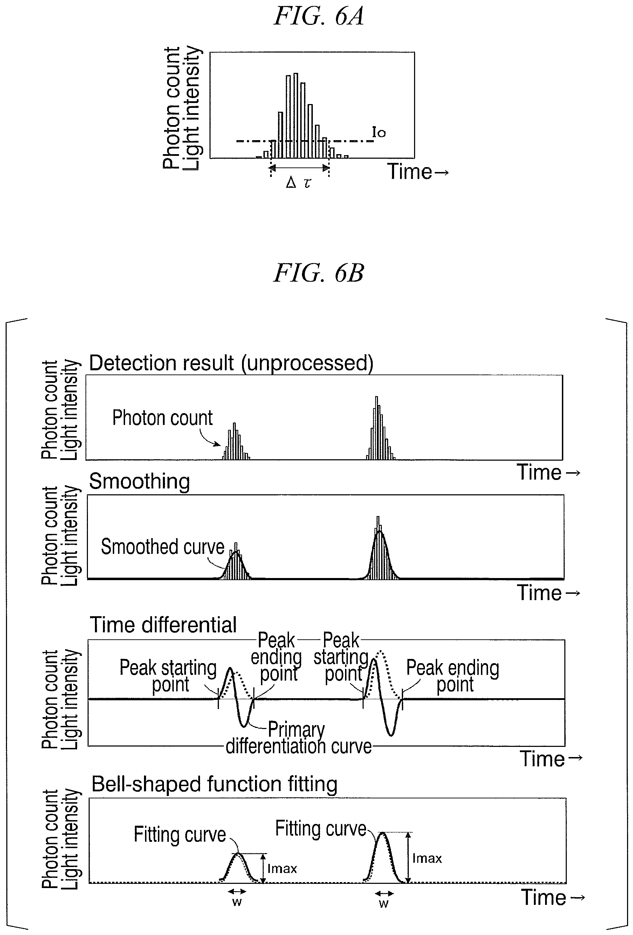

When the locus of one particle as the object of observation in its passing through the light detection region is approximately straight as shown in FIG. 4A, the change of the light intensity corresponding to the particle in the chronological light intensity data has a profile reflecting the light intensity distribution in the light detection region (light detection region determined by the optical system) (usually, an approximately bell shape) as schematically drawn in FIG. 6A. Then, in one of the methods for the detection of a particle as the object of observation, a threshold value Io is set for the light intensity. When the time width $\Delta\tau$ for which the light intensity exceeding the threshold value continues is in a predetermined range, the profile of the light intensity may be judged to correspond to one particle having passed through the light detection region. Moreover, the configuration may be such that, when the time width $\Delta\tau$ for which the light intensity exceeding the above-mentioned threshold value continues is in a predetermined range, one particle as the object of observation is detected. The threshold value Io for the light intensity and the predetermined range for the time width $\Delta\tau$ are determined based on a profile expected as the intensity of the light emitted from a combined body of a particle as the object of observation and a luminescent probe (or a luminescent probe having been freed from a particle by decomposition after being bound thereto) moving relatively to the light detection region at a predetermined speed. The concrete values of the profile may be set experimentally arbitrarily. Or, the concrete values of the profile may be selectively determined depending upon the characteristics of a combined body of a particle as the object of observation and a luminescent probe (or a luminescent probe having been freed from a particle by decomposition after being bound thereto).

Moreover, as another method of detection of the particle as the object of observation, the following method can be employed, when the light intensity distribution in the light detection region can be assumed as a Gaussian distribution:

$$I = A \cdot \exp(-2t^2/a^2) \qquad (4).$$

When the intensity A and the width a, calculated by fitting the expression (4) to the profile of a significant light intensity (a profile which can be clearly judged not to be a background), are within the respective predetermined ranges, the profile of the light intensity may be judged to correspond to one particle as the object of observation having passed through the light detection region, and thereby the detection of one particle as the object of observation will be done. Note that, when the intensity A and the width a are out of the predetermined ranges, the profile of the light intensity may be disregarded as a noise or a contaminant in the analysis.

(ii) The counting of particles as the object of observation

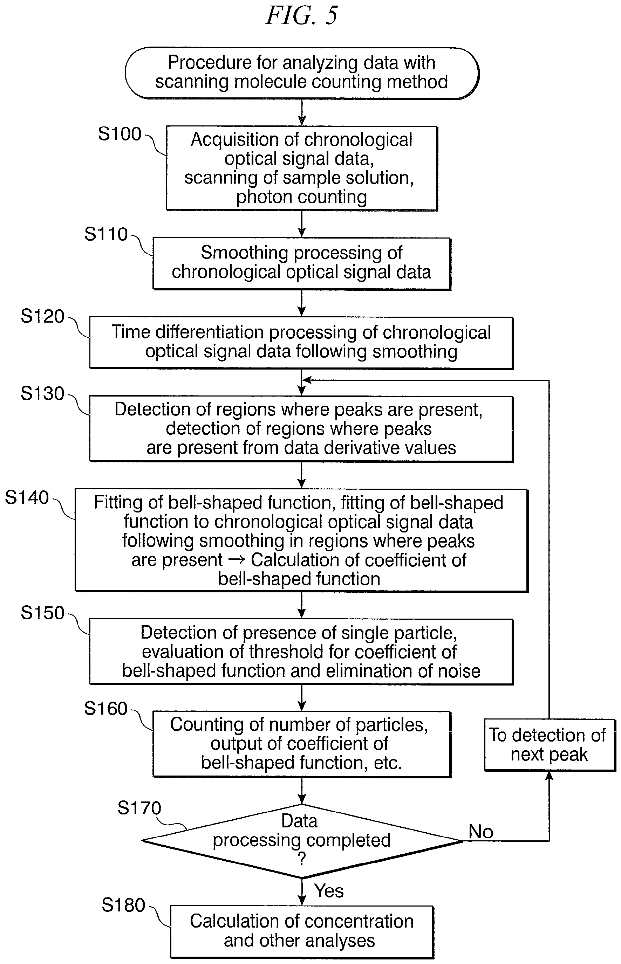

The counting of particles as the object of observation may be done by counting in an arbitrary way the number of the particles detected by the above-mentioned method of detection of the particle as the object of observation. However, for a large number of particles, for example, the counting of the particles as the object of observation may be accomplished by the processes illustrated in FIG. 5 and FIG. 6B.

As shown in FIG. 5 and FIG. 6B, in one example of ways of performing the counting of particles from the chronological light intensity (photon counts) data, after the measurement of the light intensity as explained above, that is, the scanning in the sample solution with the light detection region and the photon counting to acquire chronological optical signal data (photon count data) (S 100), a smoothing process is performed on the above-mentioned chronological optical signal data (the upper row "detection result (unprocessed)" in FIG. 6B) (S 110, mid-upper row "smoothing" in FIG. 6B). This is because the light emitted by a combined body of a particle and a luminescent probe, or a luminescent probe, is stochastic, and gaps may possibly be generated in data values in minute times. However, the above-mentioned gaps in the data values can be disregarded by the smoothing process. The smoothing process may be done, for example, by the moving average method. Note that, parameters in executing the smoothing process may be suitably set in accordance with the moving speed (scanning speed) of the position of the light detection region and/or BIN TIME in the optical signal data acquisition. The above-mentioned parameters means, for example, the number of datum points in one time of the averaging, the number of times of moving average, or the like, in the moving average method,

Next, on the chronological optical signal data after the smoothing process, in order to detect a time domain in which a significant signal exists (peak existence region), the primary differentiation value with time of the chronological optical signal data after the smoothing process is computed (S 120). As illustrated in the mid-low row "time differential" in FIG. 6B, since the change of the value increases at the time when the signal value changes in the time differential value of chronological optical signal data, the start point and the end point of a significant signal (peak signal) can be determined advantageously by referring to the above-mentioned time differential value.

After that, a significant signal (peak signal) is detected sequentially on the chronological optical signal data, and it is judged whether or not the detected peak signal is a signal corresponding to a particle as the object of observation.

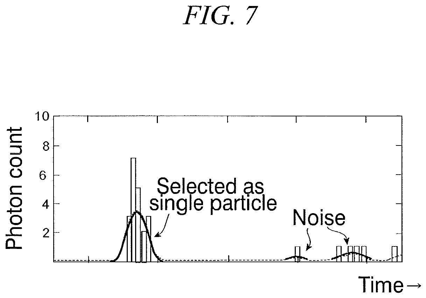

Concretely, first, on the chronological time-differential value data of the chronological optical signal data, the time differential value is sequentially referred to. Next, the starting point and the end point of one peak signal are searched and determined, by which a peak existence region is specified (S 130). When one peak existence region has been specified, the fitting of a bell-shaped function is applied to the smoothed chronological optical signal data in the peak existence region (the lower row "bell-shaped function fitting" in FIG. 6B), and parameters in the bell-shaped function, such as the peak intensity, Imax; the peak width (full width at half maximum), w; the correlation coefficient in the fitting (correlation coefficient of the least square method), etc. are calculated (S 140). Note that the bell-shaped function to be used in the fitting is typically a Gauss function. Moreover, the bell-shaped function to be used in the fitting may also be a Lorentz type function. Then, it is judged whether or not the calculated parameters of the bell-shaped function are within the respective ranges assumed for the parameters of the bell-shaped profile drawn by a light signal detected when one combined body of the particle and the luminescent probe, or one luminescent probe, passes through the light detection region (S 150). That is, in this judgment, it is judged whether or not the peak intensity, the peak width, and the correlation coefficient are respectively within the predetermined ranges. In this way, the signal, whose calculated parameters of the bell-shaped function are judged to be within the ranges assumed in a light signal corresponding to one combined body of the particle and the luminescent probe, or one luminescent probe, as shown in FIG. 7 left, is judged as a signal corresponding to one particle as the object of observation. Thereby, one particle as the object of observation has been detected, and one particle is counted (the number of particles is incremented by one: S 160). On the other hand, a peak signal whose computed parameters of the bell-shaped function are not within the assumed ranges, as shown in FIG. 7 right, is disregarded as noise.

The search and the judgment of a peak signal in the processes of the above-mentioned S 130 to S 160 are repetitively executed in the whole region of the chronological optical signal data, and whenever one particle as the object of observation is detected, it is counted as one particle. And, when the search of the peak signal in the whole region of the chronological optical signal data is completed (S 170), the count value of particles obtained till then is considered as the number of particles as the object of observation detected in the chronological optical signal data.

(iii) Determination of the number density or concentration of particles as the object of observation

When the counting of particles as the object of observation has been done, the number density or concentration of the particle as the object of observation is determined by using the whole volume of the region which the light detection region has passed through during the acquisition of the chronological optical signal data. However, the effective volume of the light detection region varies depending on the wavelength of excitation light or detected light, the numerical aperture of lenses, and the adjustment condition of the optical system, and therefore, it is generally difficult to compute the effective volume of the light detection region from the design parameter values. Accordingly, it is not easy to compute the whole volume of the region which the light detection region has passed through, either. Then, typically, the configuration may be such that the light intensity measurement, the detection of particles, and the counting thereof are performed as described above with a solution having a known concentration of the particles (reference solution) under the same condition as that for the measurement of a sample solution to be tested, so that the whole volume of the region which the light detection region has passed through, i.e., the relation between the detected number and the concentration of the particles as the object of observation, can be determined from the detected number of the particles and the concentration of the particles in the reference solution.

Preferably, the particle of the reference solution may be a light emitting label (fluorescent dye etc.) having the same emission characteristics as those of a combined body of a particle formed of the particle as the object of observation and a luminescent probe (or a luminescent probe freed from the particle as the object of observation after being bound thereto). Concretely, for example, supposing the detected number of the particles is N in a reference solution having a particle concentration C, the whole volume Vt of the region which the light detection region has passed through is given by:

$$Vt = N/C \qquad (5).$$

Alternatively, the configuration may also be such that a plurality of solutions having different concentrations are prepared as the reference solutions and the measurement is executed for each of the solutions so that the average value of the computed Vt can be employed as the whole volume Vt of the region which the light detection region has passed through. Thus, when Vt is given, the number density c of the particles of the sample solution, whose counting result of the particles is n, is given by:

$$c = n/Vt \qquad (6).$$

In this regard, the volume of the light detection region and the whole volume of the region which the light detection region has passed through are given by an arbitrary method instead of the above-mentioned method. For instance, the whole volume may be given by using FCS and FIDA. Moreover, in the photometric analysis device of this embodiment, the configuration may be such that the information on the relations (expression (5)) between the concentrations C and the numbers N of various standard particles for assumed moving patterns of the light detection region are previously stored in a storage device of the computer 18, so that a user of the device can appropriately use the stored information on the relation in conducting photometric analysis.

<Method for detecting a nucleic acid molecule>

The method for detecting a nucleic acid molecule of this embodiment is a method the object of analysis of which is a nucleic acid molecule having a specific base sequence, the method comprising: using a nucleic acid probe which can be specifically bound to the nucleic acid molecule; hybridizing the nucleic acid probe and the nucleic acid molecule to be analyzed (hereunder, may be referred to as a target nucleic acid molecule); and detecting the nucleic acid molecule to be analyzed based on the amount of the thus formed associated bodies. Furthermore, in this embodiment, the detection of the associated bodies is performed by measurement with the above-mentioned scanning molecule counting method. Since the scanning molecule counting method is a measurement method capable of measuring fluorescent particles one by one in a condition where the molecules are spaced and apart from each other, the measurement is possible for nucleic acid molecules whose concentration is relatively low in a pM order or lower. For this reason, with the method for detecting a nucleic acid molecule of this embodiment, even if the concentration of the target nucleic acid molecules in a sample solution is extremely low, the formed associated bodies can be counted with high sensitivity.

In this embodiment, the phrase "a nucleic acid probe can be specifically bound to a target nucleic acid molecule" means that the nucleic acid probe can be preferentially bound to the target nucleic acid molecule rather than other single stranded nucleic acid molecules. Specifically, when the target nucleic acid molecule is a single stranded nucleic acid molecule, it means that the nucleic acid probe can preferentially hybridize with the target nucleic acid molecule rather than other single stranded nucleic acid molecules. Moreover, when the target nucleic acid molecule is a double stranded nucleic acid molecule, it means that the nucleic acid probe can preferentially hybridize with one of the two single stranded nucleic acid molecules constituting the target nucleic acid molecule. For this reason, the nucleic acid probe may have a base sequence which is completely complementary to the base sequence of the target nucleic acid molecule, or may have a base sequence which has mismatch(es) against the base sequence of the target nucleic acid molecule.

This embodiment uses a nucleic acid probe which is specifically hybridizable with the target nucleic acid molecule. By hybridizing the target nucleic acid molecule and the nucleic acid probe, and detecting the thus formed associated bodies, the target nucleic acid molecule can be detected distinctively from other nucleic acid molecules contained in the biosample. If the target nucleic acid molecule is present in a biosample, the associated body including the nucleic acid probe is detected in a sample solution having a mixture of the biosample and the nucleic acid probe. On the other hand, if the target nucleic acid molecule is not present in the biosample, the associated body including the nucleic acid probe is not detected in the sample solution.

Note that, the nucleic acid probe for use in this embodiment may be an oligonucleotide consisting of DNA. Moreover, the nucleic acid probe may be an oligonucleotide consisting of RNA. Furthermore, the nucleic acid probe may be a chimeric oligonucleotide consisting DNA and RNA. In addition, the nucleic acid probe may be a substance a part or all of which includes a nucleotide chain or a nucleic acid analog which is capable of forming base pairs, similarly to natural nucleic acid bases. The nucleic acid analog can be exemplified by; substances in which a side chain etc. of a natural nucleotide (a nucleotide which exists in nature) such as DNA or RNA is modified with a functional group such as an amino group etc., substances labeled with a protein, a low molecular compound, etc., and the like. More concretely, the examples of the nucleic acid analog include; bridged nucleic acid (BNA), a nucleotide in which an oxygen atom at the 4' position of a natural nucleotide is substituted with a sulfur atom, a nucleotide in which a hydroxyl group at the 2' position of a natural ribonucleotide is substituted with a methoxy group, a hexitol nucleic acid (HNA), a peptide nucleic acid (PNA), and the like. Moreover, the nucleic acid to be analyzed may be DNA. Furthermore, the nucleic acid to be analyzed may be RNA. In addition, the nucleic acid to be analyzed may be artificially amplified DNA such as cDNA.

In this embodiment, by making a difference in the fluorescence intensity between the state in which the nucleic acid probe solely exists and the state in which the nucleic acid probe forms an associated body with another single stranded nucleic acid molecule, the associated body formed of the nucleic acid probe and the another single stranded nucleic acid molecule and the nucleic acid probe not forming such an associated body can be detected distinctively in the scanning molecule counting method.

Moreover, in this embodiment, a molecular beacon probe can also be used as the nucleic acid probe. The molecular beacon probe is an oligonucleotide which forms an intramolecular structure in a state of the single stranded nucleic acid molecule, and is a probe made by binding a fluorescent substance serving as an energy donor and a substance serving as an energy acceptor in FRET (a fluorescent substance and a quencher substance) so that FRET occurs in the state of the single stranded nucleic acid molecule and FRET does not occur in the state of the associated body formed by hybridization with another single stranded nucleic acid molecule. Fluorescence to be emitted from the fluorescent substance serving as an energy donor is detected from the nucleic acid probe forming the associated body. On the other hand, fluorescence to be emitted from the fluorescent substance serving as an energy donor is not detected or is attenuated, from the solely existing nucleic acid probe. Therefore, by detecting the fluorescence emitted from the fluorescent substance serving as an energy donor, the associated body including the nucleic acid probe can be detected distinctively from the solely existing nucleic acid probe.

In this embodiment, the molecular beacon probe for use as the nucleic acid probe can be exemplified by an oligonucleotide having a base sequence which is hybridizable with the target nucleic acid molecule as well as having mutually complementary base sequences in a region on the side of the 3'-end and a region on the side of the 5'-end. Preferably, the molecular beacon probe of this embodiment is bound with a fluorescent substance serving as an energy donor or a substance serving as an energy acceptor on the side of the 3'-end and bound with the other one on the side of the 5'-end, as well as having mutually complementary base sequences in regions on the side of the 3'-end and the side of the 5'-end, and forming an intramolecular structure by forming base pairs between these base sequences (a so-called stem loop structure). Note that, the mutually complementary regions which form the intramolecular base pairs of the molecular beacon probe may exist so as to interpose the region which is complementary with the target nucleic acid molecule. For example, the region on the side of the 3'-end and the region on the side of the 5'-end may respectively include the 3'-end or the 5'-end. Moreover, the region on the side of the 3'-end and the region on the side of the 5'-end may not respectively include the 3'-end or the 5'-end. In addition, the numbers of bases and the base sequences of the regions forming the base pairs may be at degrees where the stability of the formed base pairs is lower than the stability of the associated body with the nucleic acid molecule, and where the base pairs can be formed under the measurement condition.

Note that, the fluorescent substance is not particularly limited as long as it is a substance which emits fluorescence by irradiation of light of a specific wavelength, and may be used by appropriately selecting from fluorescent dyes for use in FCS, FIDA, and the like. Moreover, the labeling of the nucleic acid probe with the fluorescent substance can be performed by a usual method.

In addition, using a nucleic acid probe labeled with a fluorescent substance which causes FRET with a fluorescent double stranded nucleic acid-binding substance, it is also possible to detect the associated body including the nucleic acid probe distinguishingly from the solely existing luminescent probe. That is, either one of the fluorescent double stranded nucleic acid-binding substance or the fluorescent substance for labeling the nucleic acid probe serves as an energy donor of FRET, and the other one serves as an energy acceptor of this FRET. From the solely existing nucleic acid probe such as a first nucleic acid probe, fluorescence to be emitted from the fluorescent substance for labeling this nucleic acid probe is detected. On the other hand, since the fluorescent double stranded nucleic acid-binding substance is bound to the associated body, fluorescence to be emitted from FRET is detected from the associated body. As a result, the associated body can be detected distinctively from the solely existing nucleic acid probe.

As the fluorescent double stranded nucleic acid-binding substance which can be specifically bound to a double stranded chain structure, there can be exemplified a fluorescent intercalator, a group binder bound with a fluorescent substance, and the like. If the amount of the fluorescent intercalator to be inserted between the base pairs of the associated body is too large, the background for detecting the fluorescence emitted from FRET becomes so high that the detection precision might be influenced. For this reason, it is preferable to design the nucleic acid probe so that the region for forming the double strand in the associated body is 400 bp or shorter.

Besides, in this embodiment, the target nucleic acid molecule can also be detected by using two types of nucleic acid probes. For example, two types of nucleic acid probes are designed so that they can be hybridized specifically with the target nucleic acid molecule in mutually adjacent positions. Of these two types of specifically hybridized nucleic acid probes, one nucleic acid probe is labeled with a fluorescent substance, and the other nucleic acid probe is labeled with a fluorescent substance which causes FRET with the fluorescent substance being labeled onto the former nucleic acid probe, or a quencher substance. By respectively labeling these two types of nucleic acid probes, the associated body including the nucleic acid probes can be detected distinctively from the solely existing nucleic acid probes. That is, since these two types of nucleic acid probes are hybridized with the target nucleic acid molecule in a mutually adjacent manner, FRET occurs in the associated body consisting of these three parties. For this reason, the associated body including the nucleic acid probes can be detected by detecting fluorescence to be emitted from this FRET.

The method for detecting a nucleic acid molecule of this embodiment concretely comprises the following steps (a) to (c), and has a step for removing proteins from the following sample solution before the following step (c), or a step for removing proteins from the following biosample before the following step (a):

> (a) a step for preparing a sample solution containing a nucleic acid probe which is specifically hybridizable with a target nucleic acid molecule (nucleic acid molecule to be analyzed) and a biosample;
> (b) a step for associating the nucleic acid molecule with the nucleic acid probe in the sample solution that has been prepared in the preparing step; and
> (c) a step for, after the step (b), calculating the number of molecules of the associated bodies including the nucleic acid probe in the sample solution that has been prepared in the step (a).

Firstly, the step (a) is to prepare a sample solution containing a nucleic acid probe and a biosample. Concretely, the nucleic acid probe and the biosample are added to an appropriate solvent to prepare the sample solution. This solvent is not particularly limited as along as it is a solvent as explained below, and the solvent can be used by appropriately selecting from buffers for usual use in this technical field. That is, the solvent suffices if it does not interfere with the formation of the associated body by the nucleic acid probe, and the detection of the associated body by the scanning molecule counting method. This buffer can be exemplified by phosphate buffers such as PBS (phosphate buffered saline, pH 7.4), Tris buffer, and the like.

When a fluorescent double stranded nucleic acid-binding substance which can be specifically bound to a double stranded structure is used for the detection of the associated body including the nucleic acid probe, the fluorescent double stranded nucleic acid-binding substance is added to the sample solution, similarly to the nucleic acid probe and the like.

The biosample to be supplied in this embodiment can be exemplified by tissue pieces collected from a biological body. Specific examples can be given by: body fluids such as blood, lymph fluid, bone marrow fluid, ascitic fluid, exudative fluid, amniotic fluid, expectorated sputum, saliva, semen, bile, pancreatic fluid, and urine; tissue pieces collected from a biological body; feces, intestinal lavage fluid, lung lavage fluid, bronchial lavage fluid, bladder lavage fluid, or the like. Moreover, the biosample may be a cellular component separately collected from cells. Specific examples can be given by: a cellular component having been collected from a biological body and thereafter separately collected through a centrifugal treatment, a chromatography method, or the like; a solution in which cells have been separated by decomposing the connective tissue of a tissue piece; a cellular component having been separately collected after disrupting a tissue piece; or the like. The cellular component can be exemplified by plasma, serum, or the like, separately collected from blood. In addition, the biosample may also be a sample prepared by paraffin embedding of a tissue piece collected from a biological body, and thereafter deparaffinizing the thus produced paraffin block or a section thereof. Note that, the collection from a biological body, the centrifugal treatment, the chromatography method, the paraffin embedding, and the deparaffinization can be performed by usual methods.

The biosample may be previously applied with a proteinase treatment. The proteinase can be exemplified by proteinase K or the like. Note that, in a case of conducting a proteinase treatment as a pretreatment, usually, a treatment to inactivate the enzyme is conducted after the enzymatic reaction. For example, in a case of proteinase K, in general, the enzymatic reaction is performed at 50 to 60°C for 30 minutes to several hours, and thereafter the inactivation treatment is performed at 70°C for 5 to 15 minutes. However, in this embodiment, the inactivation treatment can be omitted.

Next, the step (b) is to associate the nucleic acid molecules in the sample solution having been prepared in the step (a). If the nucleic acid molecules in the sample solution are double stranded nucleic acid molecules, it is preferable to denature the nucleic acids before making them associate. In this embodiment, the phrase "to denature a nucleic acid molecule" means to dissociate the base pairs. For example, it means to dissociate the base pairs formed by the mutually complementary base sequences in a single stranded nucleic acid molecule to open the intramolecular structure to be in a single stranded structure, or to separate a double stranded nucleic acid molecule into single stranded nucleic acid molecules. If the nucleic acid probe is an oligonucleotide including a nucleic acid analog such as PNA, it may be possible in some cases to form the associated body including the nucleic acid probe without performing a special denaturation treatment, even if the nucleic acid molecules are double stranded nucleic acid molecules.

In this embodiment, it is preferable to perform denaturation by means of a high temperature treatment (heat denaturation) or denaturation by means of a low salt concentration treatment, because the influence to the fluorescent substance is relatively low. Particularly, it is preferable to perform heat denaturation because the manipulation is simple. Concretely, heat denaturation is capable of denaturing nucleic acid molecules in this sample solution by treating this sample solution with high temperature. Generally, the denaturation of DNA can be done by keeping the temperature at 90°C for about several seconds to two minutes. Moreover, generally, the denaturation of RNA can be done by keeping the temperature at 70°C for about several seconds to two minutes. However, the temperature for denaturing the nucleic acid molecules is multifarious depending on the base length of the target nucleic acid molecule etc., and it is not to be limited to the above-mentioned temperatures as long as the denaturation is possible. On the other hand, the denaturation by means of a low salt concentration treatment can be performed by adjustment by means of dilution with, for example, purified water or the like, so that the salt concentration of this sample solution can be sufficiently low.

In this embodiment, after the denaturation as required, the nucleic acid molecules in the sample solution are associated with each other. If the heat denaturation has been performed, the nucleic acid molecules in this sample solution can be suitably associated with each other by lowering the temperature of this sample solution to a temperature at which the nucleic acid probe and the target nucleic acid molecule can specifically hybridize with each other, after the high temperature treatment. Moreover, if the denaturation by means of a low salt concentration treatment has been performed, the nucleic acid molecules in this sample solution can be suitably associated with each other by raising the salt concentration of this sample solution to a concentration at which the nucleic acid probe and the target nucleic acid molecule can specifically hybridize with each other, by adding a salt solution or the like.

Note that, the temperature at which the nucleic acid probe and the target nucleic acid molecule can specifically hybridize with each other can be obtained from the melting curve of the associated body consisting of the target nucleic acid molecule and the nucleic acid probe. The melting curve can be obtained by, for example, changing the temperature of a solution containing only the nucleic acid probe and the target nucleic acid molecule from a high temperature to a low temperature, and measuring the absorbance or the fluorescence intensity of this solution. From the obtained melting curve, a temperature within a range from the temperature at which the denatured nucleic acid probe and target nucleic acid molecule start to form an associated body to the temperature at which almost all of them have formed associated bodies, can be set as the temperature at which the nucleic acid probe and the target nucleic acid molecule can specifically hybridize with each other. Instead of the temperature, the concentration at which the nucleic acid probe and the target nucleic acid molecule can specifically hybridize with each other can be obtained by determining the melting curve by changing the salt concentration in the solution from a low concentration to a high concentration in the same manner.

The temperature at which the nucleic acid probe and the target nucleic acid molecule can specifically hybridize with each other can be generally substituted with a Tm value (melting temperature). For example, the Tm value of the region which is hybridizable with the target nucleic acid molecule (the temperature at which 50% of double stranded DNA is dissociated into single stranded DNA) can be calculated from the base sequence information of the nucleic acid probe by using a usual primer/probe design software etc. In this embodiment, preferably, the temperature of the sample solution is lowered to about a temperature at Tm value ±3°C of the region in the nucleic acid probe having a base sequence which is complementary to the target nucleic acid molecule.

Moreover, it is preferable to relatively slowly lower the temperature of the sample solution for forming the associated body so as to suppress non-specific hybridization. For example, after denaturing the nucleic acid molecules by setting the temperature of the sample solution at 70°C or higher, the liquid temperature of this sample solution can be lowered at a cooling rate of 0.05°C/sec. or quicker.

Besides, so as to suppress non-specific hybridization, it is preferable to previously add a surfactant, formamide, dimethyl sulfoxide, urea, or the like, in the sample solution prior to the step (c). A single type of, or a combination of two or more types of, these compounds may be added. By adding these compounds, non-specific hybridization can be less likely to occur under a relatively low temperature environment. These compounds may be contained in the sample solution when preparing the sample solution in the step (a). Moreover, these compounds may also be added to the sample solution after the step (b) and before the step (c).

Various impurities such as proteins having autofluorescence contained in a biosample may be a cause of the occurrence of non-specific signals or hindering against the optical path of transmission in the measurement by the scanning molecule counting method. In this embodiment, since the measurement by the scanning molecule counting method in the step (c) is conducted in a condition where biosample-derived proteins have been removed, the influences of the biosample-derived impurities are remarkably suppressed and the target nucleic acid molecule at a low concentration can be detected with good precision.

The removal of the biosample-derived proteins may be conducted before the measurement by the scanning molecule counting method in the step (c). For example, in this embodiment, the sample solution may be prepared by mixing a biosample, to which a protein removal treatment has been applied after the collection from a biological body, with the nucleic acid probe in the step (a). Moreover, the protein removal treatment may be applied to the sample solution after the step (a) and before the step (b). In addition, the protein removal treatment may also be applied to the sample solution after the step (b) and before the step (c). Since this embodiment is capable of saving the labor and time for preparing the sample, it is preferable to apply the protein removal treatment to the sample solution in the following cases, and it is more preferable to apply the protein removal treatment to the sample solution after the step (a) and before the step (b):

a case after the step (a) and before the step (b); and
a case after the step (b) and before the step (c).

The protein removal treatment can be adopted by appropriately selecting from among methods known in this technical field. For example, the deproteinized biosample etc. can be obtained by contacting; the biosample before mixing with the nucleic acid probe, the sample solution prepared in the step (a), or the sample solution after the step (b) (hereunder, referred to as the biosample etc.), with an inorganic support so as to thereby adsorb nucleic acid molecules contained in the biosample etc. to the inorganic support, and thereafter eluting the adsorbed nucleic acid molecules from the inorganic support.

Regarding the inorganic support, a known inorganic support capable of adsorbing nucleic acid molecules can be used. Moreover, the shape of the inorganic support is not particularly limited, and it may be a particulate form or a membrane form. The inorganic support can be exemplified by: silica-containing particles (beads) of silica gel, siliceous oxide, glass, diatomite, etc.; porous membranes of nylon, polycarbonate, polyacrylate, nitrocellulose, etc.; or the like. Regarding the solvent for eluting the adsorbed nucleic acid molecules from the inorganic support, a solvent for usual use in eluting nucleic acid molecules from such a known inorganic support can be appropriately used. It is particularly preferable that the eluting solvent is purified water. Note that, it is preferable that the inorganic support having the nucleic acid molecules adsorbed thereto is washed with an appropriate washing buffer, and thereafter the nucleic acid molecules are eluted from the inorganic support.

In this embodiment, the proteins contained in the biosample etc. may be denatured before conducting the protein removal treatment. The protein denaturation can be conducted by a known method. For example, the proteins in the biosample etc. can be denatured by adding a compound usually used as a protein denaturant such as a chaotropic salt, an organic solvent, a surfactant, or the like, to the biosample etc. In the case of adding a chaotropic salt, an organic solvent, a surfactant, or the like, to the biosample etc., one type, or two or more types of compounds may be added. The chaotropic salt that can be contained in the biosample etc. can be exemplified by guanidine hydrochloride, guanidine isothiocyanate, sodium iodide, sodium perchlorate, sodium trichloroacetate, and the like. The surfactant that can be contained in the biosample etc. is preferably a nonionic surfactant. The nonionic surfactant can be exemplified by Tween 80, CHAPS (3-[3-cholamidopropyldimethylammonium]-1-propanesulfonate), Triton X-100, Tween 20, and the like. The organic solvent that can be contained in the biosample etc. is preferably a phenol. The phenol may be either neutral or acidic. In a case of using an acidic phenol, RNA can be selectively extracted in the water phase rather than DNA.

The denatured proteins can be removed, for example, by centrifugation so as to precipitate the denatured proteins, and collecting the supernatant alone. Moreover, the denatured proteins can be more entirely removed rather than simply conducting the centrifugation, by adding chloroform, sufficiently mixing them by agitation with a vortex or the like, thereafter conducting centrifugation so as to precipitate the denatured proteins, and collecting the supernatant alone. The obtained supernatant may also be applied with an additional protein removal treatment by contacting it with the above-mentioned inorganic support.

Besides, if the biosample is a type of biosample whose protein content is relatively low such as plasma, serum, or the like, proteins can be removed by aggregating the proteins using a deproteinization agent, and thereafter collecting the liquid component through a solid-liquid separation treatment. Concretely, the deproteinization agent is added to the biosample, then the mixture is agitated or left still to thereby aggregate the proteins, thereafter a centrifugation treatment is conducted, and the supernatant is collected. As the deproteinization agent, for example, an ion-exchange resin or such a highly affinitive substance to proteins can be used.

Thereafter, in this embodiment, as the step (c), the number of molecules of the associated bodies including the nucleic acid probe in the sample solution is counted by the scanning molecule counting method. Concretely, the sample solution after forming the associated bodies is set in the above-mentioned photometric analysis device for the scanning molecule counting method, and fluorescence emitted from the associated bodies is detected and analyzed by the above-mentioned technique, by which the number of molecules of the associated bodies is calculated. The calculated number of molecules of the associated bodies is the number of the target nucleic acid molecules contained in the measurement sample. That is, the target nucleic acid molecule can be detected by detecting the associated body.

Examples

Next is a more detailed description of embodiments of the present invention with reference to examples and the like. However, the embodiments of the present invention are not to be limited to the following examples and the like.

[Test Example 1

In Test Example 1, artificially synthesized oligonucleotides were used as the single stranded target nucleic acid molecule and the nucleic acid probe, samples produced by adding them to an equine whole blood or an equine plasma were used as biosample-simulating samples, and the target nucleic acid molecule in the simulated biosamples was detected by the scanning molecule counting method.

The base sequences of the target nucleic acid molecule A and the nucleic acid probe A used for the measurement are shown in Table 1. The nucleic acid probe A is the molecular beacon probe. The underlined bases are regions which are hybridized with each other when forming the intramolecular structure.

[Table 1]

| | Base sequences | |
|---|---|---|
| Nucleic acid probe A | TAMRA-5'-CCTACGCCAACAGCTCCAACTACGTAGG-3'-BHQ2 | 1 |
| Target nucleic acid molecule A | 5'-TGACTGAATATAAACTTGTGGTAGTTGGAGCTGTTGGCGTAGGCA-3' | 2 |

<Preparation of sample solution>

In Test Example 1, a 50 pM nucleic acid probe solution was prepared by mixing the target nucleic acid molecule A and the nucleic acid probe A respectively at 50 pM in Tris buffer (10 mM Tris-HCl, 1 mM EDTA, 100 mM NaCl, and pH 8.0). Moreover, a 5 pM nucleic acid probe solution was prepared by mixing the target nucleic acid molecule A and the nucleic acid probe A respectively at 5 pM in the above-mentioned Tris buffer. The obtained nucleic acid probe solutions were subjected to an annealing treatment at 95°C for 10 minutes, 70°C for 10 minutes, 60°C for 10 minutes, 50°C for 10 minutes, and 20°C for 10 minutes, to thereby form the associated body consisting of the target nucleic acid molecule A and the nucleic acid probe A.

On the other hand, a nucleic acid extraction liquid was prepared from 50 μl of equine whole blood or 50 μl of equine plasma with use of the nucleic acid extraction kit (product name: QIAamp DNA Micro kit, manufactured by QIAGEN). The nucleic acid extraction liquid corresponds to the biosample after the protein removal treatment in the embodiment of the present invention. Moreover, the equine whole blood or the equine plasma before the treatment using the nucleic acid extraction kit corresponds to the biosample before the protein removal treatment in the embodiment of the present invention.

Respectively 50 μl of the 50 pM nucleic acid probe solution or the 5 pM nucleic acid probe solution after the annealing treatment was added to the prepared nucleic acid extraction liquids, by which sample solutions were prepared (Sample preparation (+)). Moreover, respectively 50 μl of the 50 pM nucleic acid probe solution or the 5 pM nucleic acid probe solution after the annealing treatment was added to 50 μl of the equine whole blood and the 50 μl of the equine plasma, by which sample solutions were prepared (Sample preparation (-)). Furthermore, as the control, respectively 50 μl of the 50 pM nucleic acid probe solution or the 5 pM nucleic acid probe solution after the annealing treatment was added to 50 μl of the above-mentioned Tris buffer, by which sample solutions were prepared (Control).

<Counting by the scanning molecule counting method>

In Test Example 1, the number of molecules of the associated bodies consisting of the target nucleic acid molecules A and the nucleic acid probes A in each sample solution was counted by the scanning molecule counting method. Concretely, in the measurement, a single molecule fluorescence measuring apparatus MF-20 (Olympus Corporation), equipped with the optical system of a confocal fluorescence microscope and a photon counting system was used as the photometric analysis device, to obtain the chronological photon count data of the above-mentioned respective sample solutions. At this time, 543 nm laser light was used for excitation light and irradiated at 300 μW with a rotation speed of 6,000 rpm. Moreover, the detected light wavelength was set from 560 to 620 nm using a band pass filter. Regarding the signal obtained from the avalanche photodiode, BIN TIME was set to 10 μsec and the measuring time was set to 2 seconds.

The chronological data obtained by the measurement were smoothed by the Savinzky-Golay algorithm, and then peaks were detected by differentiation. Among the regions regarded as peaks, regions which were able to be approximated to the Gauss function were extracted as signals.

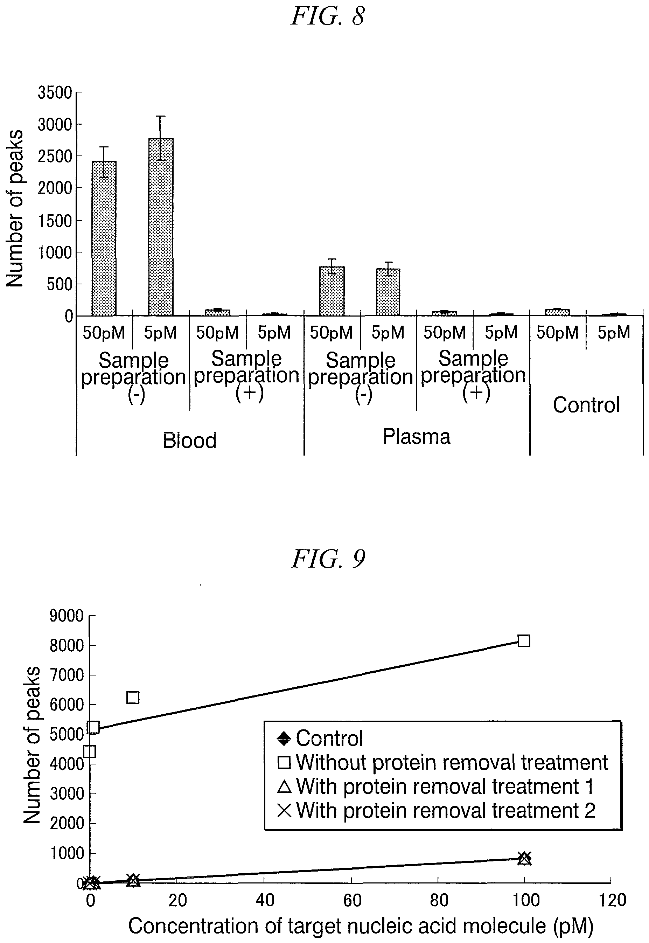

The counting results are shown in FIG. 8 and Table 2. In FIG. 8, each concentration shows the concentration of the target nucleic acid molecule A in each sample solution. The number of peaks counted in the sample solution having no biosample (Control) is the reliable counting result without being influenced by biosample-derived impurities. In the case of the sample solution having the biosample without the protein removal treatment (Sample preparation (-)), the number of peaks was remarkably larger than that of the sample solution (Control) irrespective of the number of the target nucleic acid molecules in the sample solution for both blood and plasma. This can be attributed to the impurities in the biosample which had brought about the occurrence of non-specific signals in the measurement by the scanning molecule counting method and had increased the peaks. On the other hand, in the case of the sample solution having the biosample with the protein removal treatment (Sample preparation (+)), the number of peaks equivalent to that of the sample solution having no biosample (Control) was counted. These results suggest that the biosample-derived impurities which may be a cause of the occurrence of non-specific signals in the measurement by the scanning molecule counting method can be removed in advance by performing a protein removal treatment, and more accurate measurement has become possible.

[Table 2]

|  |  |  | Concentration of target nucleic acid molecule | Number of peaks | SD |
|---|---|---|---|---|---|
| Blood | Sample preparation (-) |  | 50 pM | 2410 | 243 |
|  |  |  | 5 pM, | 2778 | 340 |
|  | Sample preparation (+) |  | 50 pM | 88 | 18 |
|  |  |  | 5 pM | 21 | 9 |
| Plasma | Sample preparation (-) |  | 50 pM | 7707 | 115 |
|  |  |  | 5 pM | 727 | 110 |
|  | Sample preparation (+) |  | 50 pM | 55 | 23 |
|  |  |  | 5 pM | 24 | 10 |
| Control |  |  | 50 pM | 97 | 2 |
|  |  |  | 5 pM | 25 | 6 |

[Example 1]

In Example 1, among PCR products obtained by PCR using a fluorescently labeled primer and a non-labeled primer, the single stranded nucleic acid molecule extended from the non-labeled primer was used as the target nucleic acid. The single stranded nucleic acid molecule extended from the fluorescently labeled primer was used as the nucleic acid probe. A sample made by adding them to a biosample obtained by deparaffinizing a paraffin-embedded tissue section was used as the simulated biosample, and the target nucleic acid molecule in a simulated biosample was detected by the scanning molecule counting method.

<Preparation of target nucleic acid molecule B and nucleic acid probe B>

In Example 1, a PCR product having a chain length of 800bp was prepared by using plasmid pUC19 (manufactured by TAKARA BIO INC.) as a template, a TAMRA-labeled oligonucleotide primer, an oligonucleotide primer not labeled with any fluorescent substance, and a PCR kit (product name: AmpliTaq Gold, manufactured by Applied Biosystems). The unreacted oligonucleotide primers were removed from the PCR product by using Wizard SV Gel and PCR Clean-Up System (manufactured by Promega Corporation). Thereafter, the presence or absence of the PCR product and the concentration thereof were measured by electrophoresis using an electrophoresis apparatus, Bioanalyzer (manufactured by Agilent Technologies, Inc.). PCR product solutions were respectively prepared by using Tris buffer (10 mM Tris-HCl, 1 mM EDTA, 100 mM NaCl, and pH 8.0) so that the concentration of the PCR product would be 2 pM, 20 pM, or 200 pM. Note that, among two single stranded nucleic acid molecules constituting the PCR product, the fluorescently-labeled nucleic acid molecule was used as the nucleic acid probe B, and the non-fluorescently-labeled nucleic acid molecule was used as the target nucleic acid molecule B.

<Preparation of sample solution>

In Example 1, 4% formalin-fixed paraffin-embedded tissue sections of rat liver and kidney were deparaffinized with xylene, and then proteinase K was added thereto to conduct an enzymatic reaction treatment at 56°C for 10 minutes. The solution after the enzymatic reaction treatment was regarded as the proteinase K-treated biosample solution.

The proteinase K-treated biosample solution was added with an equal amount of the PCR product solution of respective concentrations or the Tris buffer, by which sample solutions having PCR product concentrations at 0, 1, 10, and 100 pM were respectively prepared (without protein removal treatment). Furthermore, the respective sample solutions were subjected to an annealing treatment by heating at 95°C for 10 minutes and 55°C for 10 minutes, and then keeping at room temperature.

On the other hand, the proteinase K-treated biosample solution was additionally treated at 70°C for 10 minutes, and then was added with an equal amount of the PCR product solution or the Tris buffer, by which sample solutions having PCR product concentrations at 0, 1, 10, and 100 pM were respectively prepared. Furthermore, the respective sample solutions were subjected to an annealing treatment in the same way as that for the sample solutions (without protein removal treatment). Then, the sample solutions after the annealing treatment were purified by using a nucleic acid purification kit (product name: MinElute PCR purification kit, manufactured by QIAGEN), and the thus obtained nucleic acid extraction liquids were used as the measurement samples (with protein removal treatment 1).

In addition, the proteinase K-treated biosample solution without the treatment at 70°C for 10 minutes was added with an equal amount of the PCR product solution of respective concentrations or the Tris buffer, by which sample solutions having PCR product concentrations 0, 1, 10, and 100 pM were respectively prepared. Furthermore, the respective sample solutions were subjected to an annealing treatment in the same way as that for the sample solutions (with protein removal treatment 1) and then purified by using the nucleic acid purification kit, and the thus obtained nucleic acid extraction liquids were used as the measurement samples (with protein removal treatment 2).

As the control, the PCR product solution of respective concentrations or the Tris buffer were added at equal amounts, by which sample solutions having PCR product concentrations at 0, 1, 10, and 100 pM were respectively prepared (Control). Furthermore, the respective sample solutions were subjected to an annealing treatment in the same way as that for the sample solutions (without protein removal treatment).

<Counting by the scanning molecule counting method>

In Example 1, the number of the associated bodies consisting of the target nucleic acid molecule B and the nucleic acid probe B existing in each of the above-mentioned supernatants was counted by the scanning molecule counting method in the same manner as that of Test Example 1.

The counting results are shown in FIG. 9 and Table 3. Furthermore, in FIG. 9, the approximation straight lines regarding the relation between the concentration of the target nucleic acid molecule in each sample solution and the counted number of peaks are shown in Table 4. The number of peaks counted in the sample solution having no biosample (Control) is the reliable counting result without being influenced by biosample-derived impurities. The sample solution (without protein removal treatment) showed extremely high values as compared to those of the sample solution (Control). Moreover, the relation between the concentration of the target nucleic acid molecule in the sample solution and the detected number of peaks remarkably lacked the linearity as compared to the sample solution (Control). On the other hand, in both the sample solution (with protein removal treatment 1) and the sample solution (with protein removal treatment 2), an equivalent number of peaks to that of the sample solution (Control) were detected, showing that the target nucleic acid molecules in the measurement sample were able to be quantitatively and precisely detected in both cases. In particular, in the sample solution without the enzyme deactivation treatment after the proteinase K treatment (with protein removal treatment 2), the target nucleic acid molecules were able to be detected as precisely as the case of the sample solution (with protein removal treatment 1). Therefore, it is apparent that the method for detecting a nucleic acid molecule according to the embodiments of the present invention is capable of measurement by the scanning molecule counting method even if the enzyme deactivation treatment or the like is omitted.

[Table 3]

| | Concentration of target nucleic acid molecule | | | |
|---|---|---|---|---|
| | 0 pM | 1 pM | 10 pM | 100 pM |
| Sample solution (Control) | 9 | 13 | 75 | 784 |
| Sample solution (without protein removal treatment) | 4351 | 5177 | 6208 | 8032 |
| Sample solution (with protein removal treatment 1) | 13 | 14 | 67 | 730 |
| Sample solution (with protein removal treatment 2) | 3 | 22 | 73 | 723 |

[Table 4]

| | Approximation straight line |
|---|---|
| Sample solution (Control) | y = 7.7977x + 3.8643 R2 = 0.9998 |
| Sample solution (without protein removal treatment) | y = 30.042x + 5108.4 R2 = 0.8386 |
| Sample solution (with protein removal treatment 1) | y = 7.2396x + 5.0184 R2 = 0.9995 |
| Sample solution (with protein removal treatment 2) | y = 7.1644x + 6.4387 R2 = 0.9997 |

[Example 2]

In Example 2, among PCR products obtained by PCR using a fluorescently labeled primer and a non-labeled primer, the single stranded nucleic acid molecule extended from the non-labeled primer was used as the target nucleic acid. The single stranded nucleic acid molecule extended from the fluorescently labeled primer was used as the nucleic acid probe. A sample made by adding them to an equine plasma was used as the simulated biosample, and the target nucleic acid molecule in the simulated biosample was detected by the scanning molecule counting method.

<Preparation of target nucleic acid molecule C and nucleic acid probe C>

In Example 2, a PCR product having a chain length of 800bp was prepared by using plasmid pUC19 (manufactured by TAKARA BIO INC.) as a template, an ATTO 647N-labeled oligonucleotide primer, an oligonucleotide primer not labeled with any fluorescent substance, and a PCR kit (product name: AmpliTaq Gold, manufactured by Applied Biosystems). The unreacted oligonucleotide primers were removed from the PCR product by using Wizard SV Gel and PCR Clean-Up System (manufactured by Promega Corporation). Thereafter, the presence or absence of the PCR product and the concentration thereof were measured by electrophoresis using an electrophoresis apparatus, Bioanalyzer (manufactured by Agilent Technologies, Inc.). PCR product solutions were respectively prepared by using Tris buffer (10 mM Tris-HCl, 1 mM EDTA, 100 mM NaCl, and pH 8.0) so that the concentration of the PCR product would be 2 pM, 20 pM, or 200 pM. Note that, among two single stranded nucleic acid molecules constituting the PCR product, the fluorescently-labeled nucleic acid molecule was used as the nucleic acid probe C, and the non-fluorescently-labeled nucleic acid molecule was used as the target nucleic acid molecule C.

<Preparation of sample solution>

In Example 2, in a 1.5 ml tube were added equal amounts of the equine plasma and the PCR product solution of 2 pM, 20 pM, or 200 pM having been prepared in Example 1 or the Tris buffer, so that two 100 μl of sample solutions are prepared for each of those having PCR product concentrations at 0, 1, 10, and 100 pM respectively.

One of the thus prepared two sample solutions was added with 200 μl of a deproteinization agent (product name: BindPro (registered trademark), manufactured by Biotech Support Group Inc.) and suspended. The other one was added with 200 μl of PBS (pH 7.2) and suspended. Thereafter, each sample solution was left still for 10 minutes, and then subjected to a centrifugation treatment at 16,000 xg for 5 minutes, and the supernatant was separately collected.

<Counting by the scanning molecule counting method>

In Example 2, the number of the associated bodies consisting of the target nucleic acid molecule C and the nucleic acid probe C existing in each of the above-mentioned supernatants was counted by the scanning molecule counting method in the same manner as that of Test Example 1, except for that the 633 nm laser light was used for excitation light, and the detected light wavelength was set from 660 to 710 nm using a band pass filter.

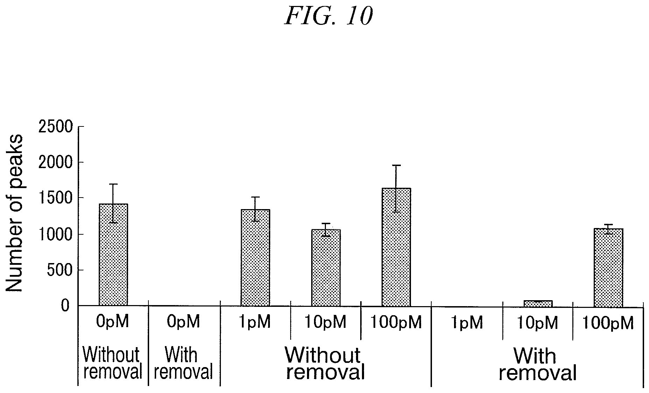

The counting results are shown in FIG. 10 and Table 5. In FIG. 10, the term "without removal" shows the results of the sample solution to which PBS had been added instead of the deproteinization agent (without protein removal treatment). Moreover, in FIG. 10, the term "with removal" shows the results of the sample solution to which the deproteinization agent had been added (with protein removal treatment).

In addition, in FIG. 10, the concentration shows the concentration of the target nucleic acid molecule in each sample solution. As a result, in the sample solution without the protein removal treatment, the number of peaks was remarkably large in all cases irrespective of the concentration of the target nucleic acid molecule. On the other hand, in the sample solution with the protein removal treatment, almost no peak was detected in the case where the concentration of the target nucleic acid molecule was 0 pM, and an increase in the number of peaks was found dependently on the concentration of the target nucleic acid molecule from 1 to 100 pM. From these results, it is apparent that, by conducting the treatment for removing biosample-derived proteins before the measurement by the scanning molecule counting method, non-specific signals are eliminated and the target nucleic acid molecule can be precisely detected.

[Table 5]

| Protein removal treatment | Concentration of target nucleic acid molecule | Number of peaks | SD |
|---|---|---|---|
| Without removal | 0 pM | 1423 | 272 |
| With removal | 0 pM | 0 | 0 |
| Without removal | 1 pM | 1348 | 166 |
| | 10 pM | 1070 | 89 |
| | 100 pM | 1647 | 325 |
| With removal | 1 pM | 3 | 1 |
| | 10 pM | 85 | 5 |
| | 100 pM | 1089 | 67 |

INDUSTRIAL APPLICABILITY

According to the method for detecting a nucleic acid molecule mentioned above, a biosample-derived nucleic acid molecule which exists only at a very low concentration in a sample solution can be detected by the scanning molecule counting method, by having them emit light with use of a probe which is specifically hybridizable with the nucleic acid molecule. For this reason, this method is applicable to fields such as a clinical test for detecting a nucleic acid molecule in a biosample.

Brief Description of the Reference Symbols

1 Photometric analysis device (confocal microscope)
2 Light source
3: Single mode optical fiber
4: Collimating lens
5: Dichroic mirror
6, 7, and 11: Reflective mirror
8: Object lens
9: Micro plate
10: Well (sample solution container)
12: Condenser lens
13: Pinhole
14: Barrier filter
15: Multi-mode optical fiber
16: Photodetector
17: Mirror deflector

17a: Stage position changing apparatus
18: Computer

**Claims**

1.  A method for detecting a nucleic acid molecule comprising:

    a step for preparing a sample solution containing (i) a biosample having the nucleic acid molecule to be detected and (ii) a nucleic acid probe which is specifically hybridizable with the nucleic acid molecule to be detected;
    a step for associating the nucleic acid molecule with the nucleic acid probe in the sample solution that has been prepared in the preparing step; and
    subsequently to the associating step, a step for calculating the number of associated bodies which comprise the nucleic acid probe hybridised with the nucleic acid molecule in the sample solution that has been prepared in the preparing step;

    whereby the method is performed by:

    a step for moving the position of a light detection region of an optical system of a confocal microscope or a multiphoton microscope in the sample solution;
    a step for detecting fluorescence emitted from the associated bodies in the light detection region while moving the position of the light detection region of the optical system in the sample solution;
    individually detecting a light signal from each of the respective associated bodies, thereby individually detecting the associated bodies; and
    the calculating step comprises a step for counting the number of the individually detected associated bodies from the detected light signals detected during the moving of the position of the light detection region; wherein proteins are removed from the sample solution before the calculating step, or proteins are removed from the biosample before the preparing step.

2.  The method for detecting a nucleic acid molecule according to claim 1, comprising a step for removing proteins from the sample solution after the associating step and before the calculating step.

3.  The method for detecting a nucleic acid molecule according to either one of claim 1 and claim 2, wherein the removing of proteins from the sample solution or the biosample is carried out by adsorbing nucleic acid molecules in the sample solution or the biosample to an inorganic support, and thereafter eluting the adsorbed nucleic acids from the inorganic support.

4.  The method for detecting a nucleic acid molecule according to any one of claim 1 to claim 3, wherein the biosample is treated with a proteinase after being collected from a biological body.

5.  The method for detecting a nucleic acid molecule according to any one of claim 1 to claim 4, wherein the nucleic acid probe is labeled with a fluorescent substance.

6.  The method for detecting a nucleic acid molecule according to any one of claim 1 to claim 4, wherein:

    the nucleic acid probe is labeled with a fluorescent substance;
    a fluorescent double stranded nucleic acid-binding substance is further contained in the sample solution in the preparing step;
    either one of the fluorescent substance labeling the nucleic acid probe and the fluorescent double stranded nucleic acid-binding substance is a fluorescent substance serving as an energy donor in a fluorescence energy transfer phenomenon, and the other one is a substance serving as an energy acceptor in the fluorescence energy transfer phenomenon; and
    in the calculating step, the fluorescence emitted from the associated body including the nucleic acid probe is fluorescence emitted from the fluorescence energy transfer phenomenon occurring between the fluorescent substance labeling the nucleic acid probe and the fluorescent double stranded nucleic acid-binding substance.

7.  The method for detecting a nucleic acid molecule according to any one of claim 1 to claim 4, wherein:

the nucleic acid probe is bound with a fluorescent substance serving as an energy donor and a substance serving as an energy acceptor so that fluorescence energy transfer occurs in a state in which the nucleic acid probe solely exists, and the fluorescence energy transfer does not occur in a state in which the nucleic acid probe forms an associated body with another single stranded nucleic acid molecule;

and the fluorescence emitted from the associated body including the nucleic acid probe is fluorescence emitted from the fluorescent substance serving as an energy donor.

8. The method for detecting a nucleic acid molecule according to any one of claim 1 to claim 7, wherein the position of the light detection region is moved at a predetermined speed, in the step for moving the position of the light detection region.

9. The method for detecting a nucleic acid molecule according to any one of claim 1 to claim 8, wherein the position of the light detection region is moved at a speed higher than the speed of diffusional movement of the associated bodies, in the step for moving the position of the light detection region.

10. The method for detecting a nucleic acid molecule according to any one of claim 1 to claim 9, wherein it is detected that one associated body has entered the light detection region, based on the shape of a chronologically detected light signal, in the step for individually detecting the associated body by individually detecting a light signal from respective associated body, from the detected light.

11. The method for detecting a nucleic acid molecule according to any one of claim 1 to claim 10, wherein the sample solution contains one or more types of substances selected from the group consisting of a surfactant, formamide, dimethyl sulfoxide, and urea.

12. The method for detecting a nucleic acid molecule according to any one of claim 1 to claim 11, wherein the associating step is performed by, after denaturing the nucleic acid molecules in the sample solution by setting the temperature of the sample solution having been prepared in the preparing step at 70°C or higher, associating the nucleic acid molecules in the sample solution by lowering the liquid temperature of the sample solution at a cooling rate of 0.05°C/sec. or quicker.

13. The method for detecting a nucleic acid molecule according to any one of claim 1 to claim 12, wherein the nucleic acid probe comprises a binding of two or more molecules selected from the group consisting of DNA, RNA, and a nucleic acid analog.

**Patentansprüche**

1. Verfahren zum Detektieren einer Nukleinsäure, umfassend:

einen Schritt des Herstellens einer Probenlösung, die (i) eine Bioprobe mit dem zu detektierenden Nukleinsäuremolekül und (ii) eine Nukleinsäuresonde, die mit dem zu detektierenden Nukleinsäuremolekül hybridisierbar ist, enthält,
einen Schritt des Assoziierens des Nukleinsäuremoleküls mit der Nukleinsäuresonde in der Probenlösung, die in dem Herstellungsschritt hergestellt worden ist; und
anschließend zum Assoziierungsschritt, einen Schritt zum Berechnen der Anzahl an assoziierten Körpern, die die mit dem Nukleinsäuremolekül in der Probenlösung, die in dem Herstellungsschritt hergestellt worden ist, hybridisierte Nukleinsäuresonde umfassen;

wobei das Verfahren durchgeführt wird durch:

einen Schritt des Bewegens der Position eines Lichtdetektierungsbereichs eines optischen Systems eines konfokalen Mikroskops oder eines Multiphotonenmikroskops in der Probenlösung;
einen Schritt zum Detektieren von von den assoziierten Körpern emittierter Fluoreszenz in dem Lichtdetektionsbereich während des Bewegens der Position des Lichtdetektionsbereichs des optischen Systems in der Probenlösung;
individuelles Detektieren eines Lichtsignals von jedem der jeweiligen assoziierten Körper,
dadurch individuelles Detektieren der assoziierten Körper; und
der Berechnungsschritt einen Schritt zum Zählen der Anzahl der individuell detektierten assoziierten Körper von den detektierten Lichtsignalen, die während des Bewegens der Position des Lichtdetektierungsbereichs detektiert wurden, umfasst;

wobei Proteine aus der Probenlösung vor dem Berechnungsschritt entfernt werden, oder Proteine aus der Bioprobe vor dem Herstellungsschritt entfernt werden.

2. Verfahren zum Detektieren eines Nukleinsäuremoleküls nach Anspruch 1, umfassend einen Schritt des Entfernens von Proteinen aus der Probenlösung nach dem Assoziierungsschritt und vor dem Berechnungsschritt.

3. Verfahren zum Detektieren eines Nukleinsäuremoleküls nach einem von Anspruch 1 oder Anspruch 2, wobei das Entfernen von Proteinen aus der Probenlösung der Bioprobe durch Adsorbieren von Nukleinsäuremolekülen in der Probenlösung oder der Bioprobe an einen anorganischen Träger durchgeführt wird, und danach Eluieren der adsorbierten Nukleinsäuremoleküle von dem anorganischen Träger.

4. Verfahren zum Detektieren eines Nukleinsäuremoleküls nach einem beliebigen von Anspruch 1 bis Anspruch 3, wobei die Bioprobe mit einer Proteinase behandelt wird, nachdem sie aus einem biologischen Körper gesammelt wurde.

5. Verfahren zum Detektieren eines Nukleinsäuremoleküls nach einem beliebigen von Anspruch 1 bis Anspruch 4, wobei die Nukleinsäuresonde mit einer Fluoreszenzsubstanz markiert ist.

6. Verfahren zum Detektieren eines Nukleinsäuremoleküls nach einem beliebigen von Anspruch 1 bis Anspruch 4, wobei:

die Nukleinsäuresonde mit einer Fluoreszenzsubstanz markiert ist;
eine fluoreszierende doppelsträngige Nukleinsäure bindende Substanz des Weiteren in der Probenlösung in dem Herstellungsschritt enthalten ist;
eine der die Nukleinsäuresonde markierenden Fluoreszenzsubstanz und der fluoreszierenden doppelsträngige Nukleinsäure bindenden Substanz eine Fluoreszenzsubstanz ist, die als Energiedonor in einem Fluoreszenz-Energie-Transfer-Phänomen dient, und die andere eine Substanz ist, die als ein Energieakzeptor in dem Fluoreszenz-Energie-Transfer-Phänomen dient; und
in dem Berechnungsschritt die von dem die Nukleinsäuresonde einschließenden, assoziierten Körper emittierte Fluoreszenz Fluoreszenz ist, die aus dem Fluoreszenz-Energie-Transfer-Phänomen emittiert wird, das zwischen der die Nukleinsäuresonde markierenden Fluoreszenzsubstanz und der fluoreszierenden doppelsträngige Nukleinsäure bindenden Substanz auftritt.

7. Verfahren zum Detektieren eines Nukleinsäuremoleküls nach einem beliebigen von Anspruch 1 bis Anspruch 4, wobei:

Die Nukleinsäuresonde mit einer Fluoreszenzsubstanz gebunden ist, die als ein Energiedonor dient, und einer Substanz, die als ein Energieakzeptor dient, so dass Fluoreszenzenergietransfer in einem Stadium stattfindet, in dem die Nukleinsäuresonde allein existiert, und der Fluoreszenzenergietransfer nicht in einem Stadium stattfindet, in dem die Nukleinsäuresonde einen assoziierten Körper mit einem anderen einzelsträngigen Nukleinsäuremolekül bildet;
und die von dem assoziierten Körper emittierte Fluoreszenz, einschließlich der Nukleinsäuresonde, Fluoreszenz ist, die von der als Energiedonor dienenden Fluoreszenzsubstanz emittiert wird.

8. Verfahren zum Detektieren eines Nukleinsäuremoleküls nach einem beliebigen von Anspruch 1 bis Anspruch 7, wobei die Position des Lichtdetektionsbereichs bei einer vorbestimmten Geschwindigkeit in dem Schritt zum Bewegen der Position des Lichtdetektionsbereichs bewegt wird.

9. Verfahren zum Detektieren eines Nukleinsäuremoleküls nach einem beliebigen von Anspruch 1 bis Anspruch 8, wobei die Position des Lichtdetektionsbereichs in dem Schritt zum Bewegen der Position des Lichtdetektionsbereichs bei einer Geschwindigkeit bewegt wird, die höher ist als die Geschwindigkeit einer Diffusionsbewegung der assoziierten Körper ist.

10. Verfahren zum Detektieren eines Nukleinsäuremoleküls nach einem beliebigen von Anspruch 1 bis Anspruch 9, wobei es detektiert wird, dass ein assoziierter Körper den Lichtdetektierungsbereich betreten hat, basierend auf der Form eines chronologisch detektierten Lichtsignals in dem Schritt zum individuellen Detektieren des assoziierten Körpers durch individuelles Detektieren eines Lichtsignals von einem jeweiligen assoziierten Körper von dem detektierten Licht.

11. Verfahren zum Detektieren eines Nukleinsäuremoleküls nach einem beliebigen von Anspruch 1 bis Anspruch 10, wobei die Probelösung eine oder mehrere Arten von Substanzen enthält, ausgewählt aus der Gruppe bestehend aus einem oberflächenaktiven Mittel, Formamid, Dimethylsulfoxid und Harnstoff.

12. Verfahren zum Detektieren eines Nukleinsäuremoleküls nach einem beliebigen von Anspruch 1 bis Anspruch 11, wobei der Assoziierungsschritt nach dem Denaturieren der Nukleinsäuremoleküle in der Probenlösung durch Einstellen der Temperatur der in dem Herstellungsschritt hergestellten Probenlösung auf 70 °C oder höher, Assoziieren der Nukleinsäuremoleküle in der Probenlösung durch Senken der Flüssigkeitstemperatur der Probenlösung bei einer Abkühlrate von 0,05 °C/Sek oder schneller durchgeführt wird.

13. Verfahren zum Detektieren eines Nukleinsäuremoleküls nach einem beliebigen von Anspruch 1 bis Anspruch 12, wobei die Nukleinsäuresonde ein Binden von zwei oder mehr Molekülen, ausgewählt aus der Gruppe bestehend aus DNA, RNA, und einem Nukleinsäureanalog umfasst.

**Revendications**

1. Procédé pour détecter une molécule d'acide nucléique comprenant :

   une étape pour préparer une solution d'échantillon contenant (i) un échantillon biologique ayant la molécule d'acide nucléique à détecter et (ii) une sonde d'acide nucléique qui peut s'hybrider spécifiquement avec la molécule d'acide nucléique à détecter ;
   une étape pour associer la molécule d'acide nucléique avec la sonde d'acide nucléique dans la solution d'échantillon qui a été préparée à l'étape de préparation ; et
   consécutivement à l'étape d'association, une étape pour calculer le nombre de corps associés qui comprennent la sonde d'acide nucléique hybridée avec la molécule d'acide nucléique dans la solution d'échantillon qui a été préparée à l'étape de préparation ;

   grâce à quoi le procédé est réalisé par :

   une étape pour déplacer la position d'une zone de détection de lumière d'un système optique d'un microscope confocal ou d'un microscope multiphotons dans la solution d'échantillon ;
   une étape pour détecter la fluorescence émise par les corps associés dans la zone de détection de lumière tout en déplaçant la position de la zone de détection de lumière du système optique dans la solution d'échantillon ;
   la détection individuelle d'un signal lumineux provenant de chacun des corps associés respectifs, détectant ainsi individuellement les corps associés ; et
   l'étape de calcul comprend une étape pour le comptage du nombre des corps associés détectés individuellement à partir des signaux lumineux détectés, détectés pendant le déplacement de la position de la zone de détection de lumière ;
   dans lequel les protéines sont éliminées de la solution d'échantillon avant l'étape de calcul, ou les protéines sont éliminées de l'échantillon biologique avant l'étape de préparation.

2. Procédé pour détecter une molécule d'acide nucléique selon la revendication 1, comprenant une étape pour éliminer les protéines de la solution d'échantillon après l'étape d'association et avant l'étape de calcul.

3. Procédé pour détecter une molécule d'acide nucléique selon l'une quelconque de la revendication 1 et la revendication 2, dans lequel l'élimination des protéines de la solution d'échantillon ou de l'échantillon biologique est réalisée par adsorption des molécules d'acide nucléique dans la solution d'échantillon ou l'échantillon biologique sur un support minéral, et l'élution par la suite des acides nucléiques adsorbés à partir du support minéral.

4. Procédé pour détecter une molécule d'acide nucléique selon l'une quelconque de la revendication 1 à la revendication 3, dans lequel l'échantillon biologique est traité avec une protéinase après avoir été recueilli à partir d'un corps biologique.

5. Procédé pour détecter une molécule d'acide nucléique selon l'une quelconque de la revendication 1 à la revendication 4, dans lequel la sonde d'acide nucléique est marquée avec une substance fluorescente.

6. Procédé pour détecter une molécule d'acide nucléique selon l'une quelconque de la revendication 1 à la revendication 4, dans lequel :

la sonde d'acide nucléique est marquée avec une substance fluorescente ;
une substance fluorescente se liant aux acides nucléiques double brin est en outre contenue dans la solution d'échantillon à l'étape de préparation ;
l'une de la substance fluorescente marquant la sonde d'acide nucléique et la substance fluorescente se liant aux acides nucléiques double brin est une substance fluorescente servant de donneur d'énergie dans un phénomène de transfert d'énergie de fluorescence, et l'autre est une substance servant d'accepteur d'énergie dans le phénomène de transfert d'énergie de fluorescence ; et
à l'étape de calcul, la fluorescence émise par le corps associé comprenant la sonde d'acide nucléique est la fluorescence émise par le phénomène de transfert d'énergie de fluorescence qui se produit entre la substance fluorescente marquant la sonde d'acide nucléique et la substance fluorescente se liant aux acides nucléiques double brin.

7. Procédé pour détecter une molécule d'acide nucléique selon l'une quelconque de la revendication 1 à la revendication 4, dans lequel :

la sonde d'acide nucléique est liée à une substance fluorescente servant de donneur d'énergie et une substance servant d'accepteur d'énergie de sorte que le transfert d'énergie de fluorescence se produit dans un état dans lequel la sonde d'acide nucléique existe isolément, et le transfert d'énergie de fluorescence ne se produit pas dans un état dans lequel la sonde d'acide nucléique forme un corps associé à une autre molécule d'acide nucléique simple brin ;
et la fluorescence émise par le corps associé comprenant la sonde d'acide nucléique est la fluorescence émise par la substance fluorescente servant de donneur d'énergie.

8. Procédé pour détecter une molécule d'acide nucléique selon l'une quelconque de la revendication 1 à la revendication 7, dans lequel la position de la zone de détection de lumière est déplacée à une vitesse prédéterminée, à l'étape pour déplacer la position de la zone de détection de lumière.

9. Procédé pour détecter une molécule d'acide nucléique selon l'une quelconque de la revendication 1 à la revendication 8, dans lequel la position de la zone de détection de lumière est déplacée à une vitesse supérieure à la vitesse de déplacement par diffusion des corps associés, à l'étape pour déplacer la position de la zone de détection de lumière.

10. Procédé pour détecter une molécule d'acide nucléique selon l'une quelconque de la revendication 1 à la revendication 9, dans lequel il est détecté qu'un corps associé est entré dans la zone de détection de lumière, sur la base de la forme d'un signal lumineux détecté chronologiquement, à l'étape pour détecter individuellement le corps associé par détection individuelle d'un signal lumineux provenant du corps associé respectif, à partir de la lumière détectée.

11. Procédé pour détecter une molécule d'acide nucléique selon l'une quelconque de la revendication 1 à la revendication 10, dans lequel la solution d'échantillon contient un ou plusieurs types de substances choisies dans le groupe constitué par un agent tensioactif, le formamide, le diméthyl sulfoxyde et l'urée.

12. Procédé pour détecter une molécule d'acide nucléique selon l'une quelconque de la revendication 1 à la revendication 11, dans lequel l'étape d'association est réalisée par, après dénaturation des molécules d'acide nucléique dans la solution d'échantillon en réglant la température de la solution d'échantillon ayant été préparée à l'étape de préparation à 70 °C ou plus, l'association des molécules d'acide nucléique dans la solution d'échantillon en abaissant la température de liquide de la solution d'échantillon à une vitesse de refroidissement de 0,05 °C/s ou plus rapide.

13. Procédé pour détecter une molécule d'acide nucléique selon l'une quelconque de la revendication 1 à la revendication 12, dans lequel la sonde d'acide nucléique comprend une liaison de deux molécules ou plus choisies dans le groupe constitué par un ADN, un ARN et un analogue d'acide nucléique.

## FIG. 1A

## FIG. 1B

## FIG. 1C

## FIG. 2A

## FIG. 2B

*FIG. 3A*

*FIG. 3B*

*FIG. 4A*

*FIG. 4B*

## FIG. 5

```
╭─────────────────────────────────────╮
│  Procedure for analyzing data with   │
│  scanning molecule counting method   │
╰─────────────────────────────────────╯
                    │
                    ▼
S100 ┌─────────────────────────────────┐
     │     Acquisition of chronological │
     │          optical signal data,   │
     │    scanning of sample solution,  │
     │          photon counting         │
     └─────────────────────────────────┘
                    │
                    ▼
S110 ┌─────────────────────────────────┐
     │       Smoothing processing of    │
     │    chronological optical signal data │
     └─────────────────────────────────┘
                    │
                    ▼
S120 ┌─────────────────────────────────┐
     │ Time differentiation processing of chronological │
     │   optical signal data following smoothing │
     └─────────────────────────────────┘
                    │
                    ▼◄──────────────────────────┐
S130 ┌─────────────────────────────────┐        │
     │ Detection of regions where peaks are present, │  │
     │    detection of regions where peaks  │     │
     │  are present from data derivative values │  │
     └─────────────────────────────────┘        │
                    │                            │
                    ▼                            │
S140 ┌─────────────────────────────────┐        │
     │ Fitting of bell-shaped function, fitting of bell-shaped │ │
     │   function to chronological optical signal data │  │
     │    following smoothing in regions where peaks │ │
     │   are present → Calculation of coefficient of │ │
     │           bell-shaped function    │        │
     └─────────────────────────────────┘        │
                    │                            │
                    ▼                            │
S150 ┌─────────────────────────────────┐        │
     │  Detection of presence of single particle, │  │
     │    evaluation of threshold for coefficient of │ │
     │  bell-shaped function and elimination of noise │ │
     └─────────────────────────────────┘        │
                    │                    ┌───────────────┐
                    ▼                    │ To detection of│
S160 ┌─────────────────────────────────┐│  next peak     │
     │    Counting of number of particles, │└───────────────┘
     │       output of coefficient of    │        ▲
     │      bell-shaped function, etc.   │        │
     └─────────────────────────────────┘        │
                    │                            │
                    ▼                            │
S170          ◇ Data ◇                           │
         ◇ processing completed ◇── No ──────────┘
              ◇     ?     ◇
                    │ Yes
                    ▼
S180 ┌─────────────────────────────────┐
     │     Calculation of concentration │
     │        and other analyses         │
     └─────────────────────────────────┘
```

## FIG. 6A

## FIG. 6B

FIG. 7

## FIG. 8

## FIG. 9

*FIG. 10*

## REFERENCES CITED IN THE DESCRIPTION

### Patent documents cited in the description

- JP 2011094293 A
- JP 2005098876 A
- JP 2008292371 A
- JP 4023523 B
- WO 2008080417 A
- JP 2007020565 A
- JP 2008116440 A
- JP H04337446 B
- JP 2965131 B
- JP 3575633 B
- JP 2006187221 A
- WO 2009158451 A1
- JP 2010044714 A

### Non-patent literature cited in the description

- **MASATAKA KINJO.** *Protein, Nucleic acid, and Enzyme,* 1999, vol. 44 (9), 1431-1438
- **MEYER-ALMS.** Fluorescence Correlation Spectroscopy. Springer, 2000, 204-224
- **NORIKO KATO et al.** *Gene & Medicine,* 2002, vol. 6 (2), 271-277